# EUROPÄISCHE PATENTSCHRIFT

(11) **EP 0 122 232 B1**
(45) Veröffentlichungstag und Bekanntmachung des Hinweises auf die Patenterteilung: **14.12.1988**
(21) Anmeldenummer: 84810179.6
(22) Anmeldetag: 10.04.1984
(51) Int. Cl.: C07J 71/00, A61K 31/58, A61K 31/585, C07J 1/00, C07J 5/00, C07J 13/00, C07J 21/00, C07J 41/00, C07J 53/00

(54) **20-Spiroxane und Analoge mit geöffnetem Ring E, Verfahren zu ihrer Herstellung, sowie pharmazeutische Präparate davon**
20-Spiroxanes and analogues with open ring E, process for their preparation and pharmaceutical compositions
20-Spiroxanes et analogues à cycle E ouvert, procédé de préparation et préparations pharmaceutiques

(30) Priorität: 13.04.1983 CH 1981/83
(43) Veröffentlichungstag der Anmeldung: 17.10.1984
(73) Patentinhaber: CIBA-GEIGY AG, 4002 Basel (CH)
(72) Erfinder: Grob, Jürgen, CH-4304 Giebenach (CH); Kalvoda, Jaroslav, Dr., CH-4102 Binningen (CH)

(56) Entgegenhaltungen:
- EP-A- 0 019 690
- EP-A- 0 061 418
- DE-A- 1 914 507
- FR-A- 2 370 755
- US-A- 3 095 412
- US-A- 3 787 396

## Beschreibung

Die Erfindung betrifft neue 20-Spiroxane und analoge Verbindungen mit geöffnetem sauerstoffhaltigen Ring E der allgemeinen Formel

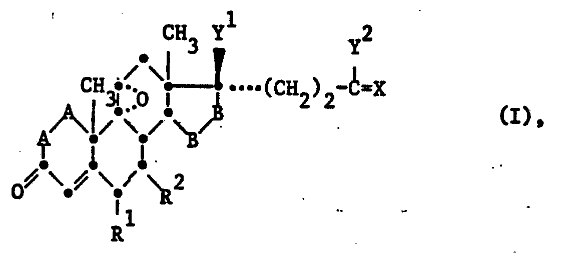

worin -A-A- die Gruppe -CH₂-CH₂- oder -CH=CH-,
R¹ Wasserstoff und
R² ein a-orientiertes Niederalkoxycarbonyl, oder
R' und R² zusammen ein a- oder β-orientierter Methylenrest,
-B-B- die Gruppe -CH₂-CH₂- oder eine a- oder β-orientierte Gruppe 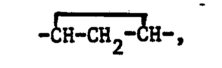 X zwei Wasserstoffatome oder Oxo,
Y¹ und Y² zusammen die Sauerstoffbrücke -0- oder
Y¹ Hydroxyl und
Y² Hydroxyl, Niederalkoxy oder, falls X gleich H₂ ist, auch Niederalkanoyloxy ist,
   sowie Salze von Verbindungen, worin X Oxo und Y² Hydroxyl ist, d. h. von entsprechenden 17ß-Hydroxy-21-carbonsäuren.

Die Erfindung betrifft auch Verfahren zur Herstellung dieser Verbindungen und pharmazeutische Zusammensetzungen enthaltend diese Verbindungen, sowie Herstellungsverfahren für solche Zusammensetzungen. Die Erfindung betrifft auch die therapeutische Verwendung der genannten Verbindungen und Zusammensetzungen, insbesondere als Aldosteron-antagonisierende Diuretika.

Wenn nicht anders angegeben, enthalten in der vorliegenden Offenbarung mit "nieder" bezeichnete organische Reste höchstens 7, vorzugsweise 1 - 4 Kohlenstoffatome.

Ein Niederalkoxycarbonylrest ist vorzugsweise ein solcher, der sich von einem Alkyl mit 1 - 4 Kohlenstoffatomen, wie Methyl, Ethyl, Propyl, Isopropyl, Butyl, Isobutyl, sec.-Butyl und tert.-Butyl ableitet; besonders bevorzugt ist Methoxycarbonyl, Ethoxycarbonyl und Isopropoxycarbonyl. Ein Niederalkoxyrest ist vorzugsweise ein solcher, der sich von einem der oben genannten C₁-C₄-Alkylreste, insbesondere einem primären, ableitet; besonders bevorzugt ist Methoxy. Ein Niederalkanoylrest ist vorzugsweise ein solcher, der sich von einem geradkettigen Alkyl mit 1 - 7 Kohlenstoffatomen ableitet; besonders bevorzugt ist Formyl und Acetyl.

Eine Methylenbrücke in 6,7- und/oder 15,16-Stellung ist vorzugsweise β-orientiert.

Bevorzugte Verbindungen der Formel I sind diejenigen, worin Y¹ und Y² zusammen die Sauerstoffbrücke -0- bedeuten.

Besonders bevorzugte Verbindungen der Formel 1 sind diejenigen, worin X Oxo bedeutet.

Unter Verbindungen der Formel I, worin R' Wasserstoff, R² Niederalkoxycarbonyl und X Oxo bedeuten, sind ganz besonders diejenigen bevorzugt, worin Y¹ zusammen mit Y² die Sauerstoffbrücke -0- darstellen.

17ß-Hydroxy-21-carbonsäuren können, wie bereits erwähnt wurde, auch in Form ihrer Salze vorliegen. In Frage kommen insbesondere Metall- und Ammoniumsalze, wie Alkalimetall- und Erdalkalimetall-, z. B. Natrium-, Calcium-, Magnesium- und vorzugsweise Kaliumsalze, bzw. Ammoniumsalze abgeleitet von Ammoniak oder einer geeigneten, vorzugsweise physiologisch verträglichen, organischen stickstoffhaltigen Base. Als Base kommen sowohl Amine, z. B. Niederalkylamine, (wie Triethylamin), Hydroxyniederalkylamine [wie 2-Hydroxyethylamin, Di-(2-hydroxyethyl)-amin oder Tri-(2-hydroxyethyl)-amin], Cycloalkylamine (wie Dicyclohexylamin) oder Benzylamine (wie Benzylamin und N,N'-Dibenzylethylendiamin), als auch stickstoffhaltige heterocyclische Verbindungen, z. B. solche aromatischen Charakters (wie Pyridin oder Chinolin) oder solche mit einem mindestens teilweise gesättigten heterocyclischen Ring (wie N-Ethylpiperidin, Morpholin, Piperazin oder N,N'-Dimethylpiperazin) in Betracht.

Zu bevorzugten Verbindungen gehören auch Alkalimetallsalze, insbesondere Kaliumsalze der Verbindungen der Formel I, worin R' und R² zusammen eine Methylengruppe oder vor allem R' Wasserstoff und R² ein Niederalkoxycarbonyl bedeuten, wobei X für Oxo und Y¹ und Y² je für Hydroxyl steht.

Besonders bevorzugte Verbindungen der Formel 1 sind z. B. die folgenden:
9α,11α-Epoxy-7α-methoxycarbonyl-20-spirox-4-en-3,21-dion,
9a,11a-Epoxy-7a-ethoxycarbonyl-20-spirox-4-en-3,21-dion,
9a,11a-Epoxy-7a-isopropoxycarbonyl-20-spirox-4-en-3,21-dion
sowie das 1,2-Dehydro-Analoge jeder von diesen Verbindungen,
9a, 11α-Epoxy-6α,7α-methylen-20-spirox-4-en-3,21-dion,
9a, 11a-Epoxy-6ß,7ß-methylen-20-spirox-4-en-3,21-dion,
9α,11α-Epoxy-6ß,7ß;15ß,16ß-bismethylen-20-spirox-4-en-3,21-dion,
sowie das 1,2-Dehydro-Analoge jeder von diesen Verbindungen,
9a,11a-Epoxy-7a-methoxycarbonyl-17ß-hydroxy-3-oxo-pregn-4-en-21-carbonsäure,
9α,11 α-Epoxy-7α-ethoxycarbonyl-17ß-hydroxy-3-oxo-pregn-4-en-21-carbonsäure,
9a,11 a-Epoxy-7a-isopropoxycarbonyl-17ß-hydroxy-3-oxo-pregn-4-en-21-carbonsäure,
9α,11α-Epoxy-17β-hydroxy-6α,7α-methylen-3-oxo-pregn-4-en-21-carbonsäure,
9α,11 α-Epoxy-17β-hydroxy-6α,7α-methylen-3-oxo-pregn-4-en-21-carbonsäure,
9α,11 α-Epoxy-17ß-hydroxy-6ß,7ß; 15ß,16ß-bismethy)en-3-oxo-pregn-4-en-21 -carbonsäure,
sowie Alkalimetallsalze, vor allem das Kaliumsalz, jeder dieser Säuren und auch ein entsprechendes 1,2-Dehydro-Analoges, jeder der genannten Carbonsäure oder eines Salzes davon,
9α,11α-Epoxy-15ß,16ß-methylen-3,21-dioxo-20-spirox-4-en-7α-carbonsäure-methylester, -ethylester und -isopropylester,
9a,11a-Epoxy-15ß,16ß-methylen-3,21-dioxo-20-spiroxa-1,4-dien-7a-carbonsäure-methylester, -ethylester und -isopropylester, sowie auch
9a,11a-Epoxy-3-oxo-20-spirox-4-en-7a-carbonsäure-methylester, -ethylester und -isopropylester,
9a,11 la-Epoxy-6ß,7ß-methylen-20-spirox-4-en-3-on,
8α,11α-Epoxy-6ß,7ß;15ß,16ß-bismethylen-20-spirox-4-en-3-on, und ferner auch
9a,1 1α-Epoxy-17ß-hydroxy-17α-(3-hydroxypropyl)-3-oxo-androst-4-en-7α-carbonsäure-methylester, -ethylester und -isopropylester,
9α,11α-Epoxy-17ß-hydroxy-17α-(3-hydroxypropyl)-6a,7a-methylen-androst-4-en-3-on,
9α,11α -Epoxy-17ß-hydroxy-17α-(3-hydroxypropyl)-6ß,7ß-methylen-androst-4-en-3-on,
9a,1 la-Epoxy-17ß-hydroxy-17a-(3-hydroxypropyl)-6ß,7ß;15ß,16ß-bis-methylen-androst-4-en-3-on, einschliesslich 17a-(3-Acetoxypropyl)- und
17a-(3-Formyloxypropyl)-Analoga der genannten Androstan-Verbindungen, sowie auch
1,2-Dehydro-Analoga aller genannten Verbindungen der Androst-4-en-3-on- und 20-Spirox-4-en-3-on-Reihe.

Die erfindungsgemässen Verbindungen zeichnen sich durch günstige biologische Eigenschaften aus und stellen somit wertvolle pharmazeutische Wirkstoffe dar. So weisen sie eine starke Aldosteron-antagonistische Wirkung auf, indem sie durch Aldosteron hervorgerufene übermässige Natrium-Retention und Kalium-Exkretion herabsetzen und normalisieren. Deshalb finden sie als Kalium-sparende Diuretika eine wichtige Anwendung in der Therapie, z. B. bei Behandlung der Hypertension, Herzinsuffizienz oder Leberzirrhose.

20-Spiroxan-Derivate mit Aldosteron-antagonisierender Wirkung sind bekannt, vgl. z. B. Fieser und Fieser: Steroids; Seite 708 (Reinhold Publ. Corp., New York, 1959) und Britische Patentschrift Nr. 1 041 534; bekannt sind auch analog wirksame 17ß-Hydroxy-21-carbonsäuren und ihre Salze, vgl. z. B. U.S. Patentschrift 3 849 404. Bisher in der Therapie angewendete Verbindungen dieser Art haben jedoch einen beträchtlichen Nachteil, indem sie immer eine gewisse sexual-spezifische Aktivität besitzen, welche sich bei der üblichen langdauernden Therapie früher oder später störend auswirkt. Besonders unerwünscht sind dabei Störungen, die auf die antiandrogene Wirksamkeit der bekannten Antialdosteron-Präparate zurückzuführen sind.

Es wurde jetzt gefunden, dass die oben charakterisierten 9a,11a-Epoxy-Verbindungen der Formel I überraschenderweise diese unerwünschten Nebenwirkungen in einem wesentlich geringeren Ausmass aufweisen, obwohl sie die günstige Antialdosteron-Wirkung von analog gebauten, aber in 9,11-Stellung nicht substituierten Verbindungen völlig beibehalten. So weist z. B. 9α,11α-Epoxy-6ß,7ß-methylen-20-spirox-4-en-3,21-dion an adrenal-ektomisierten männlichen Ratten im Kagawa-Test [Kagawa et al.: Proc. Soc. Exptl. Biol. Med. (N.Y.) 115, (1964) 837 - 840] im ganzen getesteten Dosisbereich von 1 - 10 mg/kg eine gleich grosse, wenn nicht stärkere, Aldosteron-antagonisierende Wirksamkeit als die entsprechende 9,11-unsubstituierte Vergleichssubstanz 6ß,7ß-Methylen-20-spirox-4-en-3,21-dion (J.F. Zawadzki et al.: U.S. Patent 3 849 404) auf. Dagegen aber erweist sich die erstere Verbindung in einem spezifischen quantitativen Test in vitro, in welchem als Maßstab antiandrogener Wirkung die Bindung der Testsubstanz an Androgen-Rezeptore in Homogenaten von ventraler Prostata der Ratte gemessen wird, als etwa 20-mal (nach 2-stündiger Testzeit) bis sogar 27-mal (nach 20-stündiger Testzeit) schwächer bindend als die obengenannte Vergleichsverbindung.

Die chemischen Namen von Verbindungen der Formel I und von analogen Verbindungen mit denselben charakteristischen Strukturmerkmalen werden im Sinne der geläufigen Nomenklatur folgendermassen abgeleitet:
für Verbindungem, worin Y¹ zusammen mit Y² für -0- stehen, von 20-Spiroxan (z. B. eine Verbindung der Formel 1, worin X für Oxo und Y¹ zusammen mit Y² für -0- stehen, wird von 20-Spiroxan-21-on abgeleitet);
für diejenigen, worin je Y¹ und Y² Hydroxyl und X Oxo ist, vom 17ß-Hydroxy-17a-pregnan-21-carbonsäure; und für diejenigen, worin je Y¹ und Y² Hydroxyl und X zwei Wasserstoffatome bedeutet, von 17ß-Hydroxy-17a-(3-hydroxypropyl)-androstan.

Da die cyclischen und offenkettigen Formen, d. h. Lactone bzw. 17ß-Hydroxy-21-carbonsäuren und ihre Salze, zueinander in einem so engen Verhältnis sind, dass die letzteren lediglich als eine hydratisierte Form der ersteren betrachtet werden können, sind bevor- und nachstehend, sofern nicht anders spezifisch angegeben ist, sowohl bei den Endstoffen der Formel I wie auch bei analog gebauten Ausgangsstoffen und Zwischenprodukten jeweils alle genannten Formen zusammen zu verstehen.

Die eingangs charakterisierten Verbindungen der Formel 1 können durch an sich bekannte Analogieverfahren hergestellt werden, z. B. dadurch, dass man
a) eine Verbindung der Formel 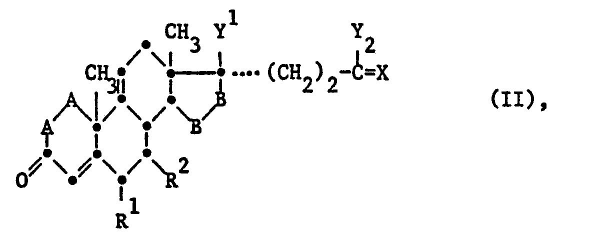 worin A-A, B-B, R¹, R², X, Y¹ und Y² die obengenannten Bedeutungen haben, mit einer Peroxysäure behandelt, oder
b) eine Verbindung der Formel 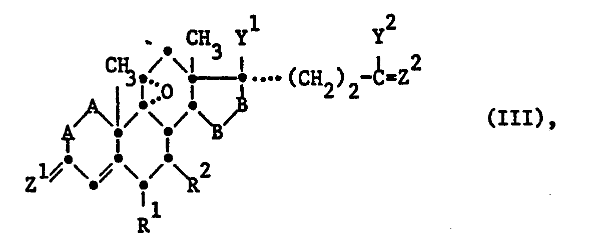 worin A-A, B-B, R¹, R², Y¹ und Y² die obengenannten Bedeutungen haben und mindestens eines der Symbole Z¹ und Z² für Hydroxyl zusammen mit Wasserstoff steht und das andere dieselbe Bedeutung hat oder für Oxo steht oder, als das Symbol Z², auch für zwei Wasserstoffatome stehen kann, mit einem Oxidationsmittel behandelt, oder
c) zur Herstellung einer Verbindung, worin Y¹ Hydroxyl und die übrigen Symbole die obengenannten Bedeutungen haben, eine Verbindung der Formel 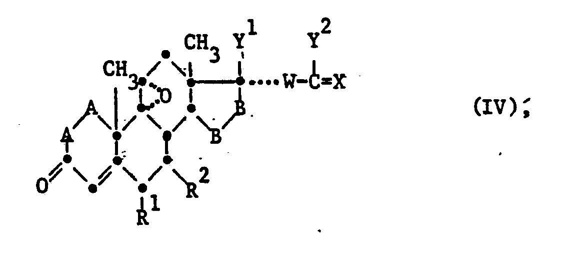 worin A-A, B-B, R¹, R², X und Y² die oben angegebenen Bedeutungen haben, Y^{l} Hydroxyl und W eine Gruppe -CH=CH- oder -C≡C- bedeutet, zur Sättigung der Mehrfachbindung in der Seitenkette hydriert, oder
d) zur Herstellung einer Verbindung der Formel I, worin Y^{l} und Y² zusammen die Sauerstoffbrücke -0- bedeuten und die übrigen Symbole die obengenannten Bedeutungen haben, eine Verbindung der Formel 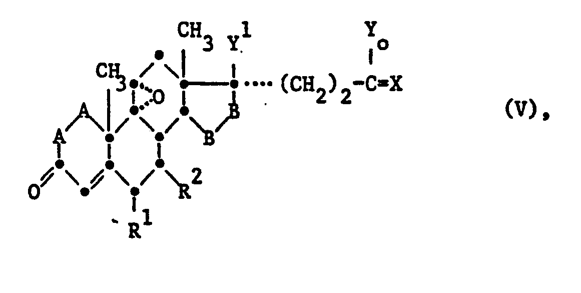 worin A-A, B-B, R¹, R² und X die obengenannten Bedeutungen haben, Y¹ Hydroxyl und Y₀ eine Abgangsgruppe bedeutet, unter Abspaltung der Gruppe Yₒ cyclisiert, oder
e) zur Herstellung einer Verbindung der Formel I, worin R¹ Wasserstoff und R² eine Niederalkoxycarbonylgruppe bedeutet und die übrigen Symbole die obengenannten Bedeutungen haben, eine Verbindung der Formel 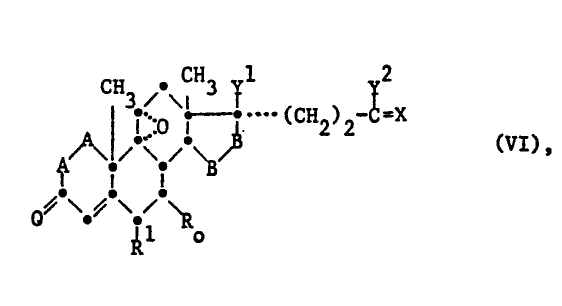 worin A-A, B-B, X, Y¹ und Y² die obengenannten Bedeutungen haben, R¹ Wasserstoff und Rₒ freies Carboxyl bedeutet, oder ein reaktionsfähiges Derivat oder Salz einer solchen Verbindung, in Ester umwandelt, oder
f) zur Herstellung einer Verbindung, worin R¹ und R² zusammen eine Methylenbrücke darstellen und die übrigen Symbole die obengenannten Bedeutungen haben, an eine Verbindung der Formel 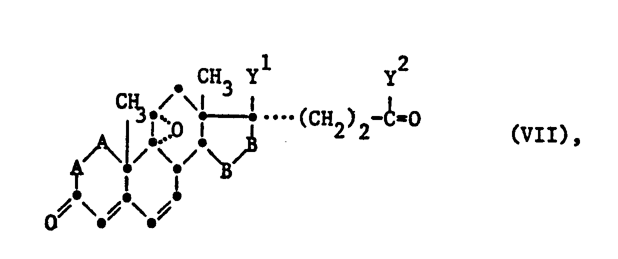 worin A-A, B-B, Y¹ und Y² die obengenannten Bedeutungen haben, die Methylengruppe anlagert, und, wenn erwünscht,
g) eine erhaltene Verbindung der Formel I, worin A-A für -CH₂-CH₂-steht, mit einem Dehydrierungsmittel zur Einführung der 1,2-Doppelbindung behandelt, und/oder
h) eine erhaltene Verbindung der Formel I, worin Y¹ und Y² je eine Hydroxylgruppe bedeutet, durch Abspaltung der Elemente von Wasser zur Verbindung der Formel I, worin Y¹ und Y² zusammen die Sauerstoffbrücke darstellen, cyclisiert, und/oder

i) eine erhaltene Verbindung der Formel I, worin X für zwei Wasserstoffatome steht, zu einer entsprechenden Verbindung, worin X für Oxo steht, oxidiert, und/oder
j) in einer erhaltenen Verbindung der Formel I, worin X zwei Wasserstoffatome bedeutet und je Y^{l} und Y² eine freie Hydroxylgruppe darstellen, die endständige Hydroxylgruppe acyliert, und/oder
k) eine erhaltene Verbindung der Formel I, worin X für Oxo, Y^{l} für Hydroxyl und Y² für Hydroxyl oder Niederalkoxy stehen, oder beide zusammen die Sauerstoffbrücke -0- darstellen, in ein Salz der entsprechenden 17ß-Hydroxy-21-carbonsäure der Formel I, worin X für Oxo und je Y^{l} und Y² für Hydroxyl stehen, überführt und/oder ein solches Salz zur freien Säure und/oder die freie Säure oder ein Salz davon zum Niederalkylester umwandelt.

Verfahrensvariante a), d. h. die Epoxidierung der 9(11)-Doppelbindung, erfolgt in an sich bekannter Weise durch das Behandeln des Ausgangsmaterials der Formel II mit einer Peroxysäure, vorzugsweise einer organischen Peroxysäure, z. B. einer aliphatischen Peroxysäure, wie insbesondere Perameisensäure oder Peressigsäure, oder vorzugsweise einer aromatischen Peroxysäure. Von den letztgenannten verwendet man vorteilhaft die Perbenzoesäure oder eine substituierte Perbenzoesäure, wie m-Chlorperbenzoesäure oder Monoperoxyphthalsäure (Perphthalsäure). Die Reaktion wird vornehmlich in einem inerten organischen Lösungsmittel ausgeführt, z. B. in einem Alkan, wie Pentan, Hexan oder Heptan, einem halogenierten Niederalkan, wie insbesondere Methylenchlorid, Chloroform oder 1,2-Dichlorethan, oder einem offenkettigen oder cyclischen Ether, wie insbesondere Diethylether, Dioxan oder Tetrahydrofuran, oder einem zweckmässigen Gemisch davon. Die Reaktionstemperatur soll in der Regel nicht eine solche übersteigen, bei welcher die spontane Zersetzung des Reaktionsmittels schneller als die Epoxidierungsreaktion verläuft, vornehmlich arbeitet man bei Zimmertemperatur oder vorzugsweise unterhalb dieser bis zu etwa -20°C, insbesondere zwischen -10 bis +10°C.

Ausgangsstoffe der Formel II, sofern sie nicht bekannt sind, können durch an sich bekannte Analogieverfahren hergestellt werden, z. B. analog einer der nachfolgend beschriebenen Verfahrensvarianten b) - k), oder deren Kombination, ausgehend von bekannten Ausgangsmaterialien, z. B. von entsprechend substituierten 17-Oxoderivaten der Androstan-Reihe durch den konventionellen Aufbau der 3-Hydroxypropyl-Seitenkette bzw. des Spiro-Rings. Alternativ kann man auch eine Verbindung, die analog einer Verbindung der Formel 1 ist, aber anstelle des 9,11-Epoxyrings eine 11α- oder 11ß-Hydroxylgruppe enthält, dehydratisieren; die 11-Hydroxyverbindung kann z. B. durch mikrobiologisches Hydroxylieren einer 9,11-unsubstituierten Verbindung erhalten werden.

Verfahrensvariante b) erfolgt ebenfalls in an sich bekannter Weise unter Anwendung von konventionellen Oxidations-Mitteln und -Verfahren, die zur Umwandlung einer Hydroxylgruppe in die Oxogruppe gebräuchlich sind. Als bevorzugte Oxidationsmittel verwendet man dabei Verbindungen des 6-wertigen Chroms, wie Chromtrioxid, Chromsäure und ihre Metallsalze, insbesondere Alkalimetallsalze, und als bevorzugtes Reaktionsmedium Niederalkancarbonsäuren, wie Essig- und Propionsäure, oder Pyridin oder Aceton, gegebenenfalls unter Verdünnung mit einem halogenierten Niederalkan, wie Dichlormethan oder Chloroform. Die Reaktionsbedingungen kann man dem spezifischen Charakter der Hydroxylgruppe im Ausgangsmaterial und der Oxogruppe im Produkt näher anpassen: für die Oxidation einer allylischen 3-Hydroxylgruppe werden milde Bedingungen, wie Kühlung zu einer Temperatur unterhalb Zimmertemperatur, z. B. auf etwa -10 bis +10°C bevorzugt; für die Oxidation zur Carboxylgruppe, sei es die freie oder die lactonisierte Carboxylgruppe, sind energischere Bedingungen zweckmässig, wie verlängerte Reaktionszeit, Reaktionstemperaturen im Bereich oder oberhalb (bis etwa 50°C) der Zimmertemperatur und/oder wässrige Schwefelsäure als Lösungsmittel für das Oxidationsmittel (z. B. in Form einer 8N-Lösung als s.g. Jones-Reagens). Alternativ kann man die Oxidation einer allylischen 3-Hydroxylgruppe auch mit Mangandioxid in einem halogenierten Niederalkan, wie Chloroform, bei Temperaturen von der Zimmertemperatur bis zur Siedetemperatur des Reaktionsgemisches, oder aber mit Aluminiumisopropylat und einem Keton, wie insbesondere Aceton oder Cyclohexanon, bei Temperaturen von der Zimmertemperatur bis zur Siedetemperatur des Gemisches, durchführen.

Ausgangsstoffe der Formel 111, sofern sie nicht bekannt sind, sind durch an sich bekannte Verfahren der Steroidchemie, z. B. durch die unter Verfahrensvarianten a) und/oder c) - k) beschriebenen Methoden, und deren zweckmässige Kombination erhältlich. So wird z. B. ein Ausgangsstoff der Formel 111, worin Z¹ für Oxo und Y^{l}, Y² und Z² je für Hydroxyl (wobei das letztgenannte Symbol zusätzlich ein Wasserstoff enthält) steht, dadurch erhalten, dass man eine entsprechende 17-Oxoverbindung unter vorübergehendem Schutz der 3-Oxogruppe mit einem Organometallderivat der Formel 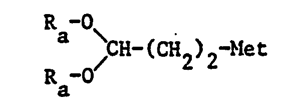 worin Met ein Alkalimetall oder die Halogenmagnesiumgruppe eines entsprechenden Grignard-Reagens und Rₐ je ein Niederalkyl oder beide zusammen CH₂-CH₂ oder Trimethylen bedeuten, umsetzt und die Oxo-Schutzgruppen abspaltet. Das erhaltene Produkt liegt als ein Gemisch von mehreren tautomeren, zum Teil auch hydratisierten Formen vor, wie sie den Teilformeln 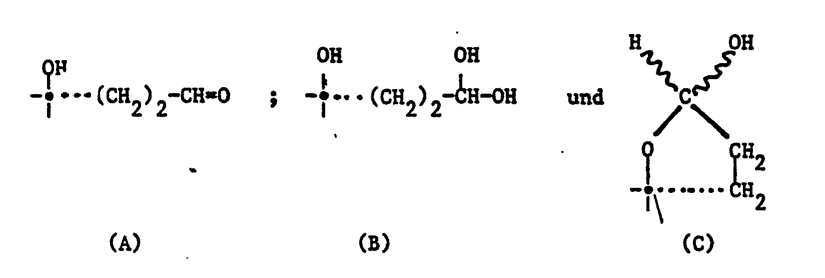 entsprechen, die sich jedoch bei Oxidation einheitlich verhalten. Verbindungen der Formel III, worin Z¹ für -OH zusammen mit Wasserstoff steht, werden erhalten, wo immer eine entsprechende 3-Oxoverbindung der Einwirkung eines üblichen Reduktionsmittels ausgesetzt wird, z. B. zugleich mit dem 21-Formyl bei Reduktion des letztgenannten 21-Carbaldehyds mit einem komplexen Hydrid.

Auch Verfahrensvariante c) erfolgt in an sich bekannter Weise unter Anwendung von konventionellen Hydrierungsmitteln unter allgemein bekannten Reaktionsbedingungen der katalytischen Hydrierung. Dabei arbeitet man mit Wasserstoffgas bei normalem oder erhöhtem Druck unter Bedingungen der heterogenen oder homogenen Katalyse. Als Katalysatore für die erstere sind fein verteilte Metalle, z. B. Raney-Metalle, wie Raney-Nickel, oder Edelmetalle, wie Palladium, Platin oder Rhodium, welche gegebenenfalls auf einem Träger, wie Calciumcarbonat oder Bariumsulphat, verteilt sind, besonders gut geeignet. Für die homogene Katalyse verwendet man insbesondere komplexe Rhodiumverbindungen, z. B. Tris-(triphenylphosphin)-rhodium(I)chlorid. Die Bedingungen sind so zu wählen, dass die 1,2- und/oder 4,5-Doppelbindung nicht mitreduziert wird.

Ausgangsstoffe der Formel IV, sofern sie nicht bekannt sind, sind durch an sich bekannte Analogieverfahren der Steroidchemie, z. B. durch die unter Verfahrensvarianten a), b) und/oder d) bis k) beschriebenen Methoden, und deren zweckmässige Kombination erhältlich. So wird z. B. ein Ausgangsstoff der Formel IV, worin W den Rest -C^{g}C- darstellt, erhalten, indem man eine geeignete 17-Oxoverbindung mit einer Ethinyl-Organometall-Verbindung, insbesondere Ethinyl-Alkalimetallverbindung, z. B. Natrium- oder Kaliumacetylid oder insbesondere Lithiumacetylid, umsetzt. Im letzten Fall ist besonders vorteilhaft, das Lithiumacetylid in Form seines Komplexes mit Ethylendiamin zu verwenden. Den eingeführten Ethinylrest kann man dann in zweiter Stufe carboxylieren, indem man in ihm das endständige Wasserstoffatom durch Behandlung mit einer Grignard-Verbindung und die nachfolgende Umsetzung des entstandenen ω-Magnesiumhalogenids mit Kohlendioxid gegen eineCarboxylgruppe austauscht. Die 3-Oxogruppe wird in der Regel bei dieser Umsetzung in der üblichen Weise geschützt. Alternativ kann man auch ein geeignetes Organometallderivat der Propiolsäure in analoger Weise einsetzen.

Rein formell, ungeachtet des Reaktionsmechanismus, erfolgt Verfahrensvariante d) durch gleichzeitiges Abspalten der Abgangsgruppe Yₒ und des Wasserstoffatoms der 17ß-Hydroxylgruppe Y^{l} unter Bildung der Sauerstoffbrücke -0-. Bei praktischer Durchführung wendet man an sich bekannte, zur Schliessung eines gesättigten Furanrings gebräuchliche Analogieverfahren der organischen Chemie an, wobei man bei der Auswahl von Reaktionsbedingungen und -mitteln jeweils spezifische Eigenschaften der Abgangsgruppe Yₒ berücksichtigt.

Eine bevorzugte Abgangsgruppe Yₒ in Verbindungen der Formel V, worin X für Oxo steht, ist eine Aminogruppe Am. Die Aminogruppe Am ist vorzugsweise eine tertiäre Aminogruppe, insbesondere eine Diniederalkylaminogruppe, wie vor allem die Dimethylamino- und Diethylaminogruppe, und bildet mit der benachbarten Carbonylgruppe eine gegebenenfalls N,Ndisubstituierte 21-Carboxamid-Gruppierung -C(=0)-Am, worin Am die obige Bedeutung hat. Die Überführung eines solchen Ausgangsstoffes in ein Lacton der Formel worin Y¹ und Y² zusammen die Sauerstoffbrucke -O- darstellen und X für Oxo steht, erfolgt in an sich bekannter Weise durch saure Mittel, vornehmlich durch die Behandlung mit einem sauren lonenaustauscher in H-Cyclus. - Der amidische Ausgangsstoff kann man in an sich bekannter Weise z. B. dadurch herstellen, das man eine entsprechende 17-Oxoverbindung unter vorübergehendem konventionellem Schutz der 3-Oxogruppe (z. B. als Ketal oder Thioketal) in Dimethylsulfoniummethylid, z. B. gemäss dem in der U.S. Patentschrift Nr. 3 320 242 beschriebenen Verfahren, umsetzt und das erhaltene 17ß,20-Epoxy-17a-methyl-steroid mit dem a-Carbanion eines N,N-Diniederalkylacetamids (bzw. einem am Methyl durch ein Alkalimetall, wie Natrium oder Lithium, metallierten N,N-Diniederalkylacetamid) in an sich bekannter Weise kondensiert.

Eine andere vorteilhafte Abgangsgruppe Yₒ, die insbesondere für Verbindungen, worin X zwei Wasserstoffatome bedeutet, geeignet ist, ist eine quaternäre Ammoniumgruppe Am+ in Form der Base; vorzugsweise ist die Gruppe Am+ eine Triniederalkylammoniumgruppe wie insbesondere die Trimethylammoniumgruppe. Die Cyclisierung der quaternären Base der Formel V, worin X für zwei Wasserstoffatome und Yₒ für Am+ OH- steht, zur Verbindung der Formel 1, worin Y^{l} und Y² zusammen die Sauerstoffbrücke darstellen, erfolgt durch Erhitzen der Base, gegebenenfalls in einem hochsiedenden organischen Lösungsmittel, wie Ethylenglykol, auf die Zersetzungstemperatur. Den entsprechenden Ausgangsstoff kann man in an sich bekannter Weise herstellen. Dazu behandelt man eine entsprechende 17-Oxoverbindung unter vorübergehendem konventionellem Schutz der 3-Oxogruppe mit einer Organometallverbindung der Formel R_{c}-(CH₂)₃-M, worin

M für eine Gruppierung MgX, in welcher X ein Halogenatom ist, oder für ein Alkalimetallatom, insbesondere ein Lithiumatom, steht, und

R_{c} eine Diniederalkylaminogruppe, vorzugsweise die Dimethylaminogruppe,

bedeutet. Eine dadurch entstandene 17ß-Hydroxy-17a-(3-diniederalkylaminopropyl)-Verbindung wird dann durch die Anlagerung eines Niederalkylesters einer starken Säure, z. B. eines Niederalkylsulfats oder Niederalkylhalogenids, wie insbesondere Methyljodids, in ein entsprechendes quaternäres Triniederalkylammoniumsalz umgewandelt. Aus diesem wird die entsprechende quaternäre Base durch Behandeln mit einer starken Base, vorzugsweise einem Metallhydroxid, z. B. Silberhydroxid oder einem Alkalimetall- oder Erdalkalimetallhydroxid, wie Kalium-, Natrium- oder Bariumhydroxid, freigesetzt.

Eine andere vorteilhafte Abgangsgruppe Y in Verbindungen der Formel V, worin X zwei Wasserstoffatome bedeutet, ist eine reaktive veresterte Hydroxylgruppe. Die Ester-bildende Komponente ist dabei z. B. eine sauerstoffhaltige anorganische Säure, wie Schwefelsäure, schweflige Säure, Phosphorsäure, phosphorige Säure oder ein Substitutionsderivat davon, in welchem eine oder mehrere der Hydroxylgruppen durch Halogen, insbesondere Chlor, ersetzt sind (d.h. ein Säurerest vom Typ CISO₂-, CISO-, C1₂P-, C1₂P(=0)- oder C1₄P-), oder eine sauerstofffreie anorganische Säure, insbesondere eine Halogenwasserstoffsäure, wie Chlor-, Brom- oder Jodwasserstoffsäure, oder aber eine starke organische Säure, z. B. Oxalsäure oder insbesondere eine Sulfonsäure, wie eine aliphatische oder aromatische carbocyclische Sulfonsäure, z. B. insbesondere Methan-, Ethan-, Trifluormethan-, bzw. Benzol-, p-Toluol-, oder p-Brombenzol-sulfonsäure. - Die Cyclisierung durch die Abspaltung einer solchen reaktiven veresterten Hydroxylgruppe erfolgt durch Behandeln mit einem anorganischen oder organischen basischen Mittel. Anorganische basische Mittel zu diesem Zweck sind z. B. Hydroxide von Alkali- und Erdalkalimetallen, wie Natrium oder Kalium bzw. Barium oder Calcium, sowie deren Salze mit schwachen anorganischen oder organischen Säuren, wie insbesondere Carbonate und Hydrocarbonate bzw. Acetate und Formiate. Organische basische Mittel zu diesem Zweck sind beispielsweise tertiäre Basen aliphatischen Charakters, wie tertiäre Amine abgeleitet von Niederalkyl- und Benzyl-Resten (z. B. Triethylamin Benzyl-dimethyl-amin, Diisopropyl-ethyl-amin, Diisopropyl-benzyl-amin Dibenzyl-methyl-amin oder Dimethyl-butyl-amin) und ihre heterocyclischen gesättigten Analoga (z. B. N-Methylpyrrolidin, N-Methylpiperidin, N-Benzylpiperidin oder N,N'-Dimethylpiperazin), oder insbesondere aromatische heterocyclische Basen, wie Pyridin und seine C-methylierten Analoga (z. B. Collidin) oder Chinolin. - Eine besonders vorteilhafte Durchführung dieser Verfahrensvariante besteht darin, das man eine im Reaktionsgemisch gebildete reaktive veresterte Hydroxylgruppe in situ abspaltet, d. h. unmittelbar danach, als sie im selben Reaktionsmedium durch die Veresterung der endständigen Hydroxylgruppe (d. h. der durch das Symbol Y² bezeichneten) in einer Verbindung der Formel I mittels eines reaktionsfähigen Säurederivats in einem basischen Medium entstanden ist. Ein reaktionsfähiges Säurederivat ist dabei insbesondere ein Säurehalogenid, wie Säurechlorid, das sich von einer der oben genannten sauerstoffhaltigen anorganischen und organischen Säuren ableitet. Als ein typisches derartiges Reaktionsmittel ist Methansulfochlorid und ganz besonders p-Toluolsulfochlorid bevorzugt, als Reaktionsmedium kommt vor allem eine aromatische heterocyclische Base, wie Pyridin, in Betracht. Auf diese Weise werden Endstoffe der Formel I, worin X für zwei H-Atome und je ^{y}' und Y² für Hydroxyl stehen, zu Endstoffen der Formel worin X für zwei H-Atome und Y' zusammen mit Y² für -0- stehen, cyclisiert.

Verfahrensvariante e) wird auch in an sich bekannter Weise durchgeführt. Üblicherweise erfolgt die Umwandlung der Säure der Formel VI in den gewünschten Ester der Formel I durch eine der zahlreichen konventionellen Veresterungsmethoden, z. B. durch Behandeln mit einem Niederalkanol oder einem reaktionsfähigen Derivat davon, gegebenenfalls in Gegenwart von Katalysatoren, insbesondere sauren Katalysatoren, und/oder wasserentziehenden Mitteln, z. B. eines symmetrisch substituierten Carbodiimids, wie insbesondere des N,N'-Dicyclohexylcarbodiimids. Unter sauren Katalysatoren kommen vor allem starke anorganische Sauren, wie Schwefel-, Phosphor- und Perchlorsäure, sowie organische Sulfonsäuren, wie Methan-, Benzol- oder p-Toluolsulfonsäure, in Betracht; der entsprechende Alkohol wird im Überschuss, meistenfalls zugleich als Lösungsmittel, genommen. Alternativ kann man auch mit einem Diazoalkan, vor allem Diazomethan, in an sich bekannter Weise verestern oder die freie Säure in ein reaktionsfähiges Derivat davon, wie ein Chlorid oder Anhydrid, z. B. gemischtes Anhydrid mit Trifluoressigsäure, umwandeln und dieses mit dem entsprechenden Niederalkanol reagieren lassen. - Ausgangsstoffe der Formel VI, sofern sie nicht bekannt sind, werden durch an sich bekannte Analogieverfahren hergestellt, z. B. durch Anlagerung von Cyanwasserstoff an die 6,7-Doppelbindung einer geeigneten 3-Oxo-4,6-dien-Verbindung (siehe unten) und eine konventionelle Umwandlung der Cyanogruppe in die Carboxylgruppe (z. B. durch Hydrolyse oder durch Reduktion zur Formylgruppe und anschliessende Oxidation).

Verfahrensvariante f) wird auch in an sich bekannter Weise unter Anwendung von konventionellen Analogieverfahren zur Anlagerung der Methylengruppe an eine Doppelbindung durchgeführt. Die Anlagerung erfolgt z. B. gemäss einer bevorzugten Variante dadurch, dass man eine 6,7-Dehydroverbindung der Formel VII mit Dimethyloxosulfoniummethylid umsetzt. Diese Variante hat auch den wesentlichen Vorteil, dass sie eine sehr hohe Stereospezifität aufweist und vorwiegend 6,7-Methylenverbindungen einer, meistens der a-Konfiguration, der Methylengruppe liefert. Die Umsetzung wird z. B. zweckmässig so ausgeführt, dass man unter einem inerten Gas, wie in der Stickstoffatmosphäre, und unter Ausschluss von Feuchtigkeit eine Mineralöl-Dispersion von Natriumhydrid mit Trimethylsulfoxoniumjodid zusammenbringt und Dimethylsulfoxid zusetzt, worauf die Bildung des Dimethyloxosulfoniummethylids stattfindet. Zu diesem in situ hergestellten Reagens wird der 6,7-ungesättigte Steroidausgangsstoff im Mol-Verhältnis (Reagens : Steroid) von etwa 1 : 1 bis 5 : 1 gegeben. Man lässt die Reaktion bei etwa Raumtemperatur ablaufen und behandelt das Reaktionsgemisch mit Wasser, wonach man das Steroid nach üblichen Methoden isoliert. Bei solchen Endstoffen, die alkali-empfindliche Gruppen, wie Lacton- oder Ester-Gruppen enthalten, ist die Zersetzung des Reaktionsgemisches zweckmässig so zu lenken, dass das pH möglichst im neutralen oder schwach sauren Bereich bleibt. Diese Methode ist auch zur Einführung der Methylengruppe in verschiedene Zwischenprodukte bei Herstellung der Ausgangsstoffe der Formeln II bis VI geeignet. - Die für diese Variante benötigten Ausgangsstoffe der Formel VII, sofern sie nicht bekannt sind, können durch an sich bekannte Analogieverfahren erhalten werden, z. B. durch die Wasserabspaltung (Dehydratisieren) der entsprechenden 11a- oder llß-Hydroxyverbindungen oder durch Dehydrieren von entsprechenden 6,7-gesättigten Zwischenprodukten.

Gemäss der gewünschtenfalls durchzuführenden Endstoff-Endstoff-Umwandlung g) werden 1,2-gesättigte Verbindungen zu den entsprechenden 1,2-Dehydroderivaten in an sich bekannter Weise dehydriert. Man kann dazu biologische Dehydrierungsverfahren anwenden, z. B. mittels der Mikroorganismen Corynebacterium simplex oder Septomyxa affinis oder ihrer Enzymsysteme dehydrieren, oder mit Selendioxid in einem organischen Lösungsmittel, z. B. tert.-Butylalkohol, behandeln. Vorzugsweise dehydriert man jedoch mit 2,3-Dichlor-5,6-dicyan-1,4-benzochinon während mehreren, z. B. 6 - 24 Stunden, und gegebenenfalls bei Siedehitze, in organischen Lösungsmitteln, z. B. aromatischen Kohlenwasserstoffen, wie Benzol oder Xylol, niederen aliphatischen Alkoholen, wie Ethanol, Propanol oder tert.-Butylalkohol, niederen aliphatischen Ketonen, wie Aceton oder 2-Butanon, aliphatischen Estern, wie Ethylacetat, oder cyclischen Ethern, wie Dioxan oder Tetrahydrofuran.

Die gewünschtenfalls durchzuführende Cyclisierung eines offenkettigen Endstoffes zu einem cyclischen gemäss Verfahrensvariante h) erfolgt auch in an sich bekannter Weise. Cyclisierung von Verbindungen der Formel 1, worin X für zwei Wasserstoffe steht, erfolgt z. B. in der bei Verfahrensvariante d) beschriebenen Weise. Endstoffe der Formel I, worin X für Oxo und je Y^{l} und Y² für Hydroxyl stehen, d. h. freie 17ß-Hydroxy-21-carbonsäuren, werden in an sich bekannter Weise cyclisiert (lactonisiert), indem man sie z. B. mit einem Wasser-abnehmenden Mittel, z. B. Essigsäureanhydrid, wasserfreiem Kupfersulfat, Molekularsieben oder Dicyclohexylcarbodiimid, in einem inerten organischen Lösungsmittel behandelt. Die Lactonisierung kann auch spontan, besonders unter sauren Bedingungen und/oder bei erhöhter Temperatur, eintreten und wird z. B. durch azeotropische Entwässerung vervollständigt.

Die gewünschtenfalls durchzuführende Oxidation der Methylengruppe im Spiro-Ring E zur Carbonylgruppe gemäss Verfahrensvariante i) erfolgt auch mittels an sich bekannter Methoden zur Oxidation eines Tetrahydrofuranrings zum entsprechenden Lactonring. Vornehmlich werden dazu Verbindungen des sechswertigen Chroms verwendet und die Reaktion wird unter analogen Bedingungen durchgeführt, wie es z. B. unter Verfahrensvariante b) für die Oxidation von entsprechenden offenkettigen Verbindungen, worin Y² für Hydroxyl und X für zwei Wasserstoffatome stehen, zu freien Carbonsäuren oben beschrieben wurde.

Die gewünschtenfalls durchzuführende Verfahrensvariante j) erfolgt gemäss allgemein bekannten üblichen Veresterungsverfahren, vorzugsweise durch Behandeln mit einer Niederalkansäure, wie insbesondere der Ameisensäure, allein, oder aber mit einem reaktionsfähigen Derivat davon, wie Anhydrid oder Halogenid, insbesondere Chlorid, vorzugsweise in Gegenwart einer organischen Base, insbesondere eines tertiären Amins, wie Triethylamin, Dimethylbenzylamin oder N,N-Dimethylanilin, einer gesättigten tertiären heterocyclischen Base, wie N-Ethylpiperidin oder N,N'-Dimethylpiperazin, oder einer aromatischen heterocyclischen Base, wie Chinolin, Collidin, Lutidin und vor allem Pyridin. Man kann auch zusätzlich von inerten aprotischen organischen Lösungsmitteln als Reaktionsmedium Gebrauch machen.

Die gewünschtenfalls durchzuführenden Umwandlungen gemäss Verfahrensvariante k) werden mittels allgemein bekannter, konventioneller Methoden durchgeführt. Erhaltene Lactone und 22-Ester kann man in an sich bekannter Weise in die entsprechenden 17ß-Hydroxy-21-carbonsäuren bzw. Salze davon z. B. dadurch umwandeln dass man sie mit einer Alkali- oder Erdalkalibase hydrolysiert, worauf man gegebenenfalls, wenn die freie Säure erwünscht ist, diese durch Ansäuern freisetzt.

Als Alkali-bzw. Erdalkalibasen verwendet man z. B. entsprechende Hydroxide, wie Natrium- und insbesondere Kaliumhydroxid, Carbonate, wie Natrium- und Kaliumcarbonat, oder Hydrogenacarbonate, wie Natrium- und Kaliumhydrogencarbonat; als Reaktionsmedium verwendet man zweckmässig Gemische von Wasser mit einem oder mehreren organischen Lösungsmitteln, vorzugsweise mit solchen, die mit Wasser mischbar sind, z. B. mit Niederalkanolen, wie Methanol, Ethanol oder Isopropylalkohol, mit cyclischen Ethern, wie Tetrahydrofuran oder Dioxan, mit Niederalkanonen, wie Aceton oder 2-Butanon, oder mit Niederalkylamiden niederaliphatischer Carbonsäuren, unter diesen insbesondere mit N,N-Dimethylformamid. Vorzugsweise verwendet man nicht mehr als eine äquivalente Menge Base und vermeidet energische Reaktionsbedingungen, welche andere Sauerstofffunktionen beeinträchtigen könnten. Wenn eine EsterGruppe in der 7-Stellung vorhanden ist, so kann sie unter den oben geschilderten milden Bedingungen in der Regel intakt beibehalten werden, da sie wesentlich langsamer als die veresterte oder lactonisierte 21-Carboxylgruppe hydrolysiert wird.

Die derart erhaltenen Alkalimetall- oder Erdalkalimetallsalze können in die entsprechenden freien 17ß-Hydroxy-21-carbonsäuren umgewandelt werden, indem eine Lösung, bzw. Suspension eines Salzes in Wasser oder einem Wasser enthaltenden organischen Lösungsmittel angesäuert wird. Die Salze können auch, z. B. mit einem Diniederalkylsulfat oder Niederalkylhalogenid, in die Ester umgewandelt werden. - Freie 17ß-Hydroxy-21-carbonsäuren kann man auch gewünschtenfalls durch Behandeln mit einer entsprechenden Base in Salze, z. B. in Ammoniumsalze und Salze organischer Basen, umwandeln, oder aber, z. B. in einer unter e) beschriebenen Weise, verestern.

Die Erfindung betrifft auch diejenigen Ausführungsformen der obigen Verfahren, bei denen man von einer auf irgendeiner Stufe als Zwischenprodukt erhältlichen Verbindung ausgeht und die fehlenden Schritte durchführt, oder bei denen ein Ausgangsstoff unter den Reaktionsbedingungen gebildet wird.

Pharmazeutische Präparate der vorliegenden Erfindung enthaltend eine Verbindung der Formel 1, oder ein Salz davon, können insbesondere zur Behandlung von Hyperaldosteronismus verschiedenster Formen verwendet werden. Sie enthalten eine wirksame Menge des Wirkstoffes allein oder im Gemisch mit anorganischen oder organischen, festen oder flüssigen, pharmazeutisch verwendbaren Trägerstoffen und, wenn erwünscht, auch mit anderen pharmakologisch bzw. therapeutisch wertvollen Stoffen, und eignen sich insbesondere zu enteralen, z. B. oralen oder rektalen, oder zur parenteralen Verabreichung.

Ohne eine nähere spezifische Angabe ist unter dem Begriff "Wirkstoff" im ganzen nachfolgenden Text eine Verbindung der Formel I, oder ein Salz davon, wie sie eingangs definiert sind, gemeint.

Die vorliegende Erfindung betrifft insbesondere pharmazeutische Zusammensetzungen enthaltend als Wirkstoff eine erfindungsgemässe Verbindung der Formel I (einschliesslich Salze) in Form einer sterilen und/oder isotonischen wässrigen Lösung, oder aber im Gemisch mit mindestens einem festen oder halbfesten Trägerstoff.

Die vorliegende Erfindung betrifft auch Arzneimittel, sowie insbesondere Arzneimittel in Form von Dosierungseinheiten, welche mindestens einender erfindungsgemässen Wirkstoffe allein oder im Gemisch mit einem oder mehreren Trägerstoffen enthalten, insbesondere solche in fester Form.

Die Erfindung betrifft insbesondere Arzneimittel in Form von Tabletten (einschliesslich Lutschtabletten, Granulen und Pastillen), Dragees, Kapseln, Pillen, Ampullen, Trockenvials oder Suppositorien enthaltend den oben definierten Wirkstoff allein oder im Gemisch mit einem oder mehreren Trägerstoffen. Als eine besondere Form dieser erfindungsgemässen pharmazeutischen Zusammensetzungen und Arzneimittel kommen auch solche in Betracht, die neben der erfindungsgemässen Aldosteron-antagonisierenden Verbindung der Formel I (einschliesslich Salze), die in diesem Zusammenhang als Komponente A bezeichnet wird, noch eine in bezug auf Elektrolyte unspezifische diuretische Komponente B enthalten.

Als solche in bezug auf Elektrolyt-Ausscheidung unspezifische diuretische Komponente B kommen herkömmliche "klassische" Diuretika oder deren Gemische in Betracht, die sowohl durch renale als auch durch extrarenale Wirkung auf Gewebe die Diurese erhöhen, insbesondere Substanzen mit hemmender Wirkung auf die Rückresorption im Tubulus, wie Saluretika oder Ethacrinsäure und deren Analoge. Insbesondere geeignet als die Elektrolyt-unspezifische Komponente B sind Benzothiadiazin-Derivate, wie Thiazide und Hydrothiazide, ferner Benzolsulfonamide, Phenoxyessigsäuren, Benzofuran-2-carbonsäuren und 2,3-Dihydro-benzofuran-2-carbonsäuren. Die Elektrolyt-unspezifische Komponente B kann aus einem einzelnen Wirkstoff oder einer zweckmässigen Kombination mehrerer Wirkstoffe bestehen, wobei die Wirkstoffe auch mehreren der genannten Stoffgruppen angehören können. - Ganz besonders kommen als Komponente B die folgenden herkömmlichen Diuretika in Betracht:
1-Oxo-3-(3-sulfamyl-4-chlor-phenyl)-3-hydroxyisoindolin,
6-Chlor-7-sulfamyl-3,4-dihydro-1,2,4-benzothiadiazin-1,1-dioxid,
3-Cyclopentyl-methyl-6-chlor-7-sulfamyl-3,4-dihydro-1,2,4-benzothiadiazin-1,1-dioxid, 4-(2-Methylenbutyryl)-2,3-dichlor-phenoxyessigsäure,
4-Thenoyl-2,3-dichlor-phenoxyessigsäure,
(1 -Oxo-2-methyl-2-phenyl-6,7-dichlor-5-indanyloxy)-essigsäure,
2-Chlor-4-furfurylamino-5-carboxybenzolsulfonamid,
2-Phenoxy-3-butylamino-5-carboxybenzolsulfonamid und
2-Phenoxy-3-[3-(1-pyrrolyl)-propyl]-5-carboxybenzolsulfonamid.

In derartigen erfindungsgemässen pharmazeutischen Zusammensetzungen und Arzneimitteln beträgt das Verhältnis der Komponente A zur Komponente B, bezogen auf die jeweilige mittlere effektive Dosis, etwa 4 : 1 bis etwa 1 : 4, vorzugsweise etwa 3 : 2 bis etwa 2 : 3. Da die mittlere effektive Dosis jeder spezifischen Komponente ein bekannter, oder durch bekannte pharmakologische Testmethoden einfach feststellbarer Wert ist, ist dem Fachmann leicht möglich, innerhalb der oben genannten Grenzen ein geeignetes Verhältnis beider Komponenten jedem Patienten gemäss seinem spezifischen Leiden, allgemeinen Gesundheitszustand, individueller Ansprechbarkeit und Alter, sowie auch dessen Geschlecht, zu verordnen.

Beispielsweise enthalten derartige Kombinationspräparate pro Dosiseinheit 5 bis 150 mg, insbesondere 10 bis 50 mg einer Verbindung der Formel I oder eines Salzes davon als Komponente A und, als Komponente B, beispielsweise 10 - 100 mg, insbesondere 25 - 50 mg 2-Chlor-5-[3-hydroxy-1-oxo-isoindolyl-(3)]-benzolsulfonamid oder 4-(2-Methylen-butyryl)-2,3-dichlorphenoxy-essigsäure, 5 - 50 mg, insbesondere 12 - 25 mg 6-Chlor-7-sulfamyl-3,4-dihydro-1,2,4-benzothiadiazin-1,1-dioxid oder 2-Chlor-4-furfurylamino-5-carboxybenzolsulfonamid, 2 - 20 mg, insbesondere 5 - 10 mg 2-Phenoxy-3-[3-(1-pyrrolyl)-propyl]-5-carboxybenzolsulfonamid, 0,1 - 1,0 mg, insbesondere 0,25 - 0,5 mg 3-Cyclopentylmethyl-6-chlor-7-sulfamyl-3,4-dihydro-1,2,4-benzothiadiazin-1,1-dioxid oder 2-Phenoxy-3-butylamino-5-carboxybenzolsulfonamid, 100 - 400 mg, insbesondere 200 mg 4-Thenoyl-2,3-dichlor-phenoxyessigsäure und 5 - 25 mg, insbesondere 10 mg racemische (1-Oxo-2-methyl-2-phenyl-6,7-dichlor-5-indanyloxy)-essigsäure, oder eine halbe Menge der laevo-Form dieser Säure.

- Für die Ödembehandlung in einem mittelschweren Fall werden beispielsweise 1 - 3 Dosiseinheiten täglich genommen, die die Wirkstoffe in Gewichtsmengen enthalten, welche an der höheren Grenze der oben erwähnten besonders bevorzugten Dosierung liegen; ein mittelschwerer Fall der essentiellen Hypertonie wird z. B. mit 1 - 3 Dosiseinheiten behandelt, deren Wirkstoffgehalt an der unteren besonders bevorzugten Mengengrenze liegt.

Der Ausdruck "Arzneimittel" ist angewendet zur Bezeichnung von einzelnen abgetrennten Portionen einheitlicher Zusammensetzung, welche für die medizinische Verabreichung geeignet sind. Der Ausdruck "Arzneimittel in Form von Dosierungseinheiten" ist in dieser Beschreibung angewendet zur Bezeichnung von einzelnen abgetrennten Portionen einheitlicher Zusammensetzung, welche für die medizinische Verabreichung geeignet sind und je einzeln eine spezifische Menge des erfindungsgemässen Wirkstoffes enthalten, die etwa 0,05 bis etwa 2, vorzugsweise etwa 0,1 bis etwa 1 Tagesdosis entspricht.

Die Trägerstoffe zur Verwendung in den pharmazeutischen Zusammensetzungen sind allgemein bekannte Materialien.

Die erfindungsgemässen pharmazeutischen Zusammensetzungen enthalten vorzugsweise von etwa 0,1 bis zu etwa 99,5, insbesondere von etwa 1 bis zu etwa 90 Gewichts-% des Wirkstoffes.

Die empfohlene Tagesdosis des Wirkstoffes der Formel I (einschliesslich Salze) für einen 75 kg schweren Warmblüter beträgt etwa 5 - 200 mg, vorzugsweise etwa 10 -100 mg, sie kann jedoch innerhalb weiten Grenzen in Abhängigkeit von Species, Alter und der individuellen Ansprechbarkeit variieren und die obere Mengengrösse überschreiten.

Die Herstellung der obgenannten erfindungsgemässen pharmazeutischen Zusammensetzungen, Präparaten, Arzneimitteln und Arzneimitteln in Form von Dosierungseinheiten erfolgt mittels konventioneller, an sich bekannter Herstellungsverfahren der pharmazeutischen Industrie, z. B. mittels üblicher Misch-, Granulier-, Tablettier-, Dragier-, Lösungs- und Lyophilisierungsverfahren, wobei gewünschtenfalls unter keimfreien Bedingungen gearbeitet wird oder ein Zwischenprodukt oder ein fertiges Produkt sterilisiert wird.

Die vorliegende Erfindung betrifft auch die Anwendung der Verbindungen der Formel I (einschliesslich Salze) zur Bekämpfung von verschiedensten Formen des Hyperaldosteronismus im Mensch und anderen Warmblütern, sowie eine entsprechende therapeutische Methode, die durch die Verabreichung einer wirksamen Dosis mindestens eines der erfindungsgemässen Wirkstoffe allein oder zusammen mit einem oder mehreren Trägerstoffen oder in einer Arzneimittelform charakterisiert ist. Die erfindungsgemässen Wirkstoffe werden dabei enteral, z. B. rektal oder vor allem oral, oder parenteral, wie insbesondere intravenös, verabreicht. Eine besondere Anwendung der erfindungsgemässen Verbindungen ist dadurch gekennzeichnet, dass man eine erfindungsgemässe Verbindung der Formel I, oder ein Salz davon, als die Aldosteron-antagonisierende Steroid-Komponente A, und eine in bezug auf die Elektrolytausscheidung unspezifische diuretische Komponente (Komponente B) gleichzeitig bzw. gemeinsam, insbesondere in Form einer entsprechenden pharmazeutischen Zusammensetzung oder eines Arzneimittels, verabreicht.

In den folgenden Beispielen, welche die Erfindung weiter illustrieren, ohne sie dabei einzuschränken, sind die Temperaturen in Celsiusgraden angegeben. Alle Schmelzpunkte sind unkorrigiert.

### Beispiel 1

Eine Lösung von 100 mg 7a-Methoxycarbonyl-20-spiroxa-4,9(11)-dien-3,21-dion in 2 ml Methylenchlorid wird mit 75 mg 90-%-iger m-Chlorperbenzoesäure versetzt und 18 Stunden bei ca. 4° und anschliessend noch 7 Stunden bei Zimmertemperatur stehen gelassen. Nach dem Verdünnen mit Methylenchlorid wird das Gemisch nacheinander mit 10-%-iger Kaliumjodidlösung, 10-%-iger Natriumthiosulfatlösung, eiskalter gesättigter Natriumhydrogencarbonatlösung und Wasser gewaschen, getrocknet und im Wasserstrahlvakuum eingedampft. Das amorphe Rohprodukt wird durch präparative Dünnschichtchromatographie an Kieselgel im System Methylenchlorid-Aceton (85 : 15) getrennt. Die Hauptzone wird mit 100 ml Ethylacetat eluiert und eingedampft. Das resultierende kristalline 9α,11α-Epoxy-7α-methoxycarbonyl-20-spirox-4-en-3,21-dion wird aus Methylenchlorid/Ether umgelöst, Smp. 239 - 2410.

In analoger Weise wird 7α-Methoxycarbonyl-15ß,16ß-methylen-20-spiroxa-4.9(11)-dien-3,21-dion zum 9a,11a-Epoxy-7a-methoxycarbonyl-15ß,16ß-methylen-20-spirox-4-en-3,21-dion umgewandelt.

Der Ausgangsstoff kann folgendermassen hergestellt werden:
a) Eine eiskalte Lösung von 0,636 ml wasserfreier Blausäure in 6,44 ml Benzol wird unter Feuchtigkeitsausschluss innert 1,5 St. unter Eiskühlung und Rühren mit einer Lösung von 3,54 ml Triethylaluminium in 9,66 ml Benzol versetzt und anschliessend 16 Stunden bei Zimmertemperatur rühren gelassen. Die erhaltene Diethylaluminiumcyanid-Lösung wird zu einer Lösung von 2,0 g 20-Spiroxa-4,6,9(11)-trien-3,21-dion [vgl. J. Med. Chem., 6, (1963) 732 - 735] in 40 ml Tetrahydrofuran zugegeben, 30 Minuten unter Rückfluss erhitzt und abgekühlt. Die Reaktionslösung wird unter Rühren in 40 ml 1 N Natriumhydroxid-Lösung gegossen und zweimal mit Ethylacetat extrahiert. Die organische Phase wird nacheinander mit gesättigter Kochsalzlösung und eiskalter verdünnter Salzsäure gewaschen, getrocknet und im Wasserstrahlvakuum eingedampft. Das erhaltene ölige Rohprodukt liefert nach Chromatographie an Kieselgel durch Eluieren mit einem Gemisch von Hexan-Ether-Methanol (2 :9 : 1) 7a-Cyano-20-spiroxa-4,9(11)-dien-3,21-dion, Smp. 241 - 243°.
   In analoger Weise wird 15ß,16ß-Methylen-20-spiroxa-4,6,9(11)-trien-3,21-dion (siehe Beispiel 7, Herstellung der Ausgangsstoffe) zum 7a-Cyano-15ß,16ß-methyten-20-spiroxa-4,9(11)-dien-3,21-dion umgewandelt.
b) 400 mg 7a-Cyano-20-spiroxa-4,9(11)-dien-3,21-dion aus Stufe a) werden in 16 ml Benzol gelöst, bei einer Temperatur von 10° mit 4,8 ml einer 20-%-igen (G/V)-Lösung von Diisobutyl-aluminiumhydrid in Toluol versetzt und 30 Minuten unter Eiskühlung gerührt. Das Gemisch wird auf Zimmertemperatur erwärmt, weitere 10 Minuten gerührt, mit 8,0 ml Benzol verdünnt und nochmals 20 Minuten bei Zimmertemperatur weitergerührt. Unter Kühlung mit Eis-Kochsalz werden zum Reaktionsgemisch bei einer Innentemperatur von max. 10° zuerst 4,8 ml Ethylalkohol und anschliessend 48 ml Wasser zugetropft, und das Gemisch wird während 5 Stunden unter Rückfluss erhitzt und anschliessend abgekühlt. Das Gemisch wird mit eiskalter verdünnter Salzsäure angesäuert, mit Chloroform extrahiert und die organische Phase im Wasserstrahlvakuum eingedampft.
   In analoger Weise erhält man aus 7a-Cyano-15ß,16ß-methylen-20-spiroxa-4,9(11)-dien-3,21-dion das entsprechende 7a-Formyl-15ß,16ß-methylen-20-spiroxa-4,9(11)-dien-3,21-dion.
c) Das rohe 7a-Formyl-20-spiroxa-4,9(11)-dien-3,21-dion aus Stufe b) wird in 20 ml Aceton gelöst, bei einer Temperatur von 7 - 10° mit 1,2 ml einer 8-N Lösung von Chromtrioxid in wässriger Schwefelsäure versetzt und 1 Stunde unter Eiskühlung gerührt. Das Gemisch wird mit Eiswasser verdünnt, mit Chloroform extrahiert und die organische Phase einmal mit Wasser gewaschen und zweimal mit je 40 ml gesättigter Natriumhydrogencarbonat-Lösung extrahiert. Die vereinigten alkalischen Auszüge werden unter Eiskühlung mit 4-N Salzsäure angesäuert und 10 Minuten stehen gelassen. Aus dem milchigen Gemisch wird das Produkt in Chloroform aufgenommen und nach Trocknen im Wasserstrahlvakuum eingedampft.
   Die resultierende amorphe 3,21-Dioxo-20-spiroxa-4,9(11)-dien-7a-carbonsäure wird ohne zusätzliches Reinigen weiterverarbeitet.
   In analoger Weise erhält man aus 7a-Formyl-15ß,16ß-methylen-20-spiroxa-4,9(11)-dien-3,21-dion die 3,21-Dioxo-15ß,16ß-methylen-20-spiroxa-4,9(1 1)-dien-7α-carbonsäure.

d) Eine etherische Diazomethanlösung wird zu einer Lösung von 235 mg 3,21-Dioxo-20-spiroxa-4,9(11)-dien-7a-carbonsäure in 2,35 ml Methylenchlorid solange zugetropft, bis die Entwicklung von Stickstoff aufhört. Nach 20 Minuten bei Zimmertemperatur wird die gelbe Reaktionslösung vorsichtig eingedampft und der Rückstand einmal aus Methylenchlorid-Ether-Petrolether umgelöst, wodurch 7a-Methoxycarbonyt-20-spiroxa-4,9(11)-dien-3,21-dion, Smp. 205 - 206°, resultiert.
   In analoger Weise, ausgehend von der 3,21-Dioxo-15ß,16ß-methylen-20-spiroxa-4,9(11)-dien-7a-carbonsäure, erhält man ihren entsprechenden Methylester.

### Beispiel 2

Ein Gemisch von 3,9 g 7a-Methoxycarbonyl-20-spiroxa-4,9(11)-dien-3,21-dion, 1,95 g Dikaliumhydrogenphosphat, 5,85 ml Trichloracetonitril, 17,5 30-%-iger wässriger Wasserstoffperoxidlösung und 89 ml Methylenchlorid wird bei Raumtemperatur 2 Stunden intensiv gerührt, mit weiterem Methylenchlorid verdünnt, nacheindander mit 10-%-iger Kaliumjodidlösung, 10-%-iger Natriumthiosulfatlösung, eiskalter gesättigter Natriumhydrogencarbonatlösung und Wasser gewaschen, getrocknet und im Wasserstrahlvakuum eingedampft. Das amorphe Rohprodukt wird durch präparative Dünnschichtchromatographie an Kieselgel im System Methylenchlorid-Aceton (85 : 15) getrennt. Die Hauptzone wird mit 100 ml Ethylacetat eluiert und eingedampft. Das resultierende kristalline 9a,11a-Epoxy-7a-methoxycarbonyt-20-spirox-4-en-3,21-dion vom Smp. 239-241° ist mit dem Produkt des Beispiels 1 identisch.

### Beispiel 3

a) Eine Suspension von 13,0 g 3,21-Dioxo-20-spiroxa-4,9(11)-dien-7a-carbonsäure (vgl. Beispiel 1c), 5,2 ml 1,5-Diazabicyclo(5,4,0)undec-5-en und 9,5 ml Isopropylbromid in 80 ml Benzol wird 3 Stunden unter Rückfluss gerührt. Nach Abkühlen wird das Reaktionsgemisch mit 800 ml gesättigter NaCI-Lösung versetzt und zweimal mit Ethylacetat extrahiert. Die organischen Phasen werden nacheinander mit verdünnter Salzsäure, verdünnter Natronlauge und gesättigter NaCI-Lösung gewaschen, getrocknet und eingedampft. Das Rohprodukt wird in Chloroform an 30-facher Gewichtsmenge Kieselgel chromatographiert. Die einheitlichen Fraktionen ergeben nach Umlösen aus Methylenchlorid/Ether den 3,21-Dioxo-20-spiroxa-4,9(11)-dien-7a-carbonsäureisopropylester, Smp. 138 - -139°.
b) Eine Lösung von 0,2 g 3,21-Dioxo-20-spiroxa-4,9(11)-dien-7a-carbonsäureisopropylester in 1,6 ml Methylenchlorid und 0,4 ml Trichloracetonitril wird mit 0,5 ml einer Lösung von 1,1 g Dikaliumhydrogenphosphat in 2,5 ml 30-%-iger Wasserstoffperoxid-Lösung versetzt und 5 Stunden bei 40° gerührt. Nach erneuter Zugabe von 0,4 ml 30-%-iger Wasserstoffperoxid-Lösung wird 21 Stunden bei 40° weitergerührt. Die übliche Aufarbeitung liefert ein amorphes Rohprodukt, das an 50-facher Gewichtsmenge Kieselgel im System Methylenchlorid-Aceton (98 : 2) chromatographiert wird. Durch Kristallisation der einheitlichen Fraktionen aus Methylenchlorid-Ether erhält man das 9a,11a-Epoxy-7a-isopropoxycarbonyl-20- spirox-4-en-3,21-dion, Smp. 207 - 209°.

### Beispiel 4

a) Eine Suspension von 7,2 g 3,21-Dioxo-20-spiroxa-4,9(11)-dien-7a-carbonsäure (vgl. Beispiel 1c), 2,88 ml 1,5-Diazabicyclo(5,4,0)undec-5-en und 5,6 ml Ethylbromid in 43 ml Benzol wird 3 Stunden unter Rückfluss gerührt. Nach Abkühlen wird das Reaktionsgemisch mit 400 ml gesättigter NaCI-Lösung versetzt und zweimal mit Ethylacetat extrahiert. Die organischen Phasen werden nacheinander mit verdünnter Salzsäure, verdünnter Natronlauge und gesättigter NaCI-Lösung gewaschen, getrocknet und eingedampft. Das Rohprodukt wird in Chloroform an 30-facher Gewichtsmenge Kieselgel chromatographiert. Die einheitlichen Fraktionen ergeben nach Umlösen aus Methylenchlorid/Ether den 3,21-Dioxo-20-spiroxa-4,9(11)-dien-7a-carbonsäureethylester, Smp.128 -129°.
b) Ein Gemisch von 1,93 g 3,21-Dioxo-20-spiroxa-4,9(11)-dien-7a-carbonsäureethylester, 19,3 ml Methylenchlorid, 2,89 ml Trichloracetonitril, 365 ml Dikaliumhydrogenphosphat und 8,68 ml 30-%-iger Wasserstoffperoxid-Lösung wird 6 Stunden bei 40° gerührt. Die übliche Aufarbeitung liefert ein amorphes Rohprodukt, das an 50-facher Gewichtsmenge Kieselgel im System Methylenchlorid-Aceton (95 : 5) chromatographiert wird. Durch Kristallisation der einheitlichen Fraktionen aus Methylenchlorid-Ether erhält man das 9α,11α-Epoxy-7α-ethoxycarbonyl-20-spirox-4-en-3,21-dion, Smp. 177 -179°.

### Beispiel 5

Eine Lösung von 570 mg 3,21-Dioxo-20-spiroxa-4,9(11)-dien-7a-carbonsäure (siehe Beispiel 1c) in 11,4 ml Methylenchlorid wird mit 430 mg 90-%-iger m-Chlorperbenzoesäure versetzt und 3 Stunden bei Zimmertemperatur stehen gelassen. Unter Eiskühlung wird zu diesem Gemisch eine etherische Diazomethanlösung solange zugegeben, bis keine Stickstoffentwicklung mehr sichtbar ist. Die mit Methylenchlorid verdünnte Reaktionslösung wird nacheinander mit einer 10-%-iger Kaliumjodid-Lösung, 10-%- iger Natriumthiosulfat-Lösung und eiskalter gesättigter Natriumhydrogencarbonat-Lösung gewaschen, getrocknet und im Wasserstrahlvakuum eingedampft. Das gelartige Rohprodukt wird an der 100-fachen Gewichtsmenge Kieselgel mit einem Gemisch von Methylenchlorid-Aceton (96 : 4) chromatographiert. Das erhaltene 9a,11a-Epoxy-7a-methoxycarbonyl-20-spirox-4-en-3,21-dion ist mit dem Produkt von Beispiel 1 identisch, Smp. 239 - 241 (nach zweimaligem Umlösen aus Methylenchlorid-Ether).

### Beispiel 6

Eine Lösung von 980 mg 3,21-Dioxo-20-spiroxa-4,9(11)-dien-7a-carbonsäure (vgl. Beispiel 1c) und 735 mg 90- %-iger m-Chlorperbenzoesäure in 19,6 ml Methylenchlorid wird 3 Stunden bei Zimmertemperatur stehen gelassen. Nach Verdünnen mit Methylenchlorid wird das Gemisch nacheinander mit 10-%-iger Kaliumjodid- Lösung und 10-%-iger Natriumthiosulfat-Losung gewaschen und mit einer eiskalten 0,5-N NatriumcarbonatLösung extrahiert. Die wässrige Phase wird mit Ether gewaschen und gefriergetrocknet. Das pulverige Natriumsalz der 7a-Carbonsäure wird in 4,9 ml Dimethylformamid suspendiert, mit 1,9 ml Isopropyljodid versetzt und 16 Stunden bei 40° gerührt. Das abgekühlte Reaktionsgemisch wird mit Eiswasser verdünnt und mit verdünnter Salzsäure angesäuert. Der ausgefallene Niederschlag wird abgenutscht, mit Wasser nachgewaschen und in Methylenchlorid gelöst; die Lösung wird nach Trocknen im Wasserstrahlvakuum eingedampft. Das ölige Rohprodukt ergibt nach Lösen in Methylenchlorid, Filtrieren durch Aluminiumoxid (neutral) und Eindampfen das amorphe 9α,11α-Epoxy-7α-isopropoxycarbonyl-20-spirox-4-en-3,21-dion, welches nach Umlösen aus Methylenchlorid-Ether bei 207 - 209° schmilzt.

### Beispiel 7

Eine Lösung von 15,7 g 6ß,7ß-Methylen-20-spiroxa-4,9(11)-dien-3,21-dion in 628 ml Chloroform wird mit 11,3 g 80-%-iger p-Nitroperbenzoesäure versetzt und 2 Stunden bei Zimmertemperatur stehen gelassen. Nach Verdünnen mit Methylenchlorid wird das Gemisch nacheinander je einmal mit 10-%-iger Kaliumjodid-Lösung, 10-%-iger Natriumthiosulfat-Lösung und mit eiskalter verdünnter Natronlauge gewaschen, und die organische Phase nach Trocknen im Wasserstrahlvakuum eingedampft. Durch Chromatographie auf einer 150-fachen Gewichtsmenge Kieselgel und Elution mit einem Gemisch von Toluol-Ethylacetat (80 : 20) wird das 9a,11a-Epoxy-6ß,7ß-methylen-20-spirox-4-en-3,21-dion erhalten, das nach einmaligem Umlösen aus Methylenchlorid- Ether den Schmelzpunkt von 299 - 301° aufweist.

In analoger Weise kann 6ß,7ß;15ß16ß-Bis-methylen-20-spiroxa-4,9(11)-dien-3,21-dion zum 9α,11α-Epoxy-6ß,7ß;15ß 16ß-bis-methylen-20-spirox-4-en-3,21-dion umgewandelt werden.

Die benötigten Ausgangsstoffe können folgendermassen hergestellt werden:
Unter strengem Ausschluss von Feuchtigkeit wird ein Gemisch von 30,4 g Trimethylsulfoxoniumjodid und 102 ml Dimethylsulfoxid mit 4,37 g einer 72-%-igen (G/V) Suspension von Natriumhydrid in Mineralöl versetzt und 1 Stunde bei Zimmertemperatur gerührt. Dieses Gemisch wird mit 7,80 g 20-Spiroxa-4,6,9(11)-trien-3,21-dion [vg1. J. Med. Chem., 6, (1963) 732-735] versetzt und nach dem Nachspülen mit 7,8 ml Dimethylsulfoxid 2 Stunden bei Zimmertemperatur gerührt. Unter gutem Rühren wird das Reaktionsgemisch auf Eiswasser gegossen, mit verdünnter Salzsäure angesäuert und zweimal mit Ethylacetat extrahiert. Die organische Phase wird nacheinander mit einer gesättigten Kochsalz-Lösung, eiskalter verdünnter Natronlauge und nochmals mit der Kochsalz-Lösung gewaschen, getrocknet und im Wasserstrahlvakuum eingedampft. Das Rohprodukt wird anschliessend an einer 50-fachen Gewichtsmenge Kieselgel chromatographiert und mit einem Gemisch von Toluol-Ethylacetat (85 : 15) eluiert Nach Abdampfen der Lösungsmittel resultiert 6ß,7ß-Methylen-20-spiroxa-4,9(11)-dien-3,21-dion, Smp. 174 -178° (nach doppeltem Umlösen aus Methylenchlorid-Ether).

In analoger Weise wird auch 6ß,7ß;15ß,16ß-Bis-methylen-20-spiroxa-4,9(11)-dien-3,21-dion ausgehend vom 15ß,16ß-Methylen-20-spiroxa-4,6,9(11)-trien-3,21-dion hergestellt; das letztere kann folgendermassen erhalten werden:
a) Eine Lösung von 20 g 17a,20;20,21-Bis-methylendioxy-pregn-5-en-3ß,llß-diol (U.S. Patent 3409 610) in 150 ml Pyridin und 150 g Acetanhydrid wird 1 Stunde unter Rückfluss erhitzt. Die abgekühlte Reaktionslösung wird auf 3000 g Eisflocken unter Rühren gegossen und bis zum Auftauen weiter gerührt. Der Niederschlag wird abgenutscht und an der Luft getrocknet, das rohe 3ß,11ß-Diacetoxy-17a,20;20,21-bis-methytendioxy-pregn-5-en wird ohne Reinigung weiterverarbeitet.
b) 20,3 g des luftgetrockneten 3,11-Diacetats wird unter Aussenkühlung mit Eiswasser unter Rühren portionsweise in 71 ml einer Lösung eingetragen, die durch Einleiten, bei ca. 0°, von 141 g gasförmigem Fluorwasserstoff in eine Lösung bestehend aus 100 ml lsopropylalkohol, 48 g Harnstoff und 9,6 ml Wasser vorher zubereitet wird.
   Das Reaktionsgemisch wird unter der Eiswasserkühlung 1 Stunde gerührt, vorsichtig in eine eiskalte Lösung von 142 g Natriumsulfit in 1015 ml Wasser gegossen und 20 Minuten gerührt. Das Gemisch wird mit Ethylacetat extrahiert und nacheinander mit gesättigter Natriumchloridlösung, eiskalter verdünnter Salzsäure, eiskalter verdünnter Natronlauge und wieder mit verdünnter Kochsalzlösung gewaschen, getrocknet und im Wasserstrahlvakuum eingedampft. Der Rückstand wird an einer 10-fachen Gewichtsmenge Kieselgel chromatographiert. Durch Elution mit einem Gemisch von Methylenchlorid-Aceton (95 : 5) werden einheitliche Fraktionen erhalten, die nach einmaligem Umlösen aus Methylenchlorid-Methanol-Ether 3ß,11ß-Diacetoxy-17a,21-dihydroxy-pregn-5-en-20-on vom Schmelzpunkt 231 - 233° liefern.

c) Eine Lösung von 13,8 g der letzgenannten Verbindung in 207 ml Dioxan wird mit 69 g feinpulverigen Mangandioxid versetzt und 3 Stunden am Rückfluss gekocht. Nach Abkühlen auf Zimmertemperatur wird der feste Anteil durch Abnutschen entfernt und mit Chloroform gut nachgewaschen. Das Filtrat wird eingedampft, in Methylenchlorid gelöst und durch eine 10-fache Gewichtsmenge neutrales Aluminiumoxid filtriert. Durch Abdampfen des Lösungsmittels wird kristallines 3ß,11ß-Diacetoxy-androst-5-en-17-on erhalten, das nach einmaligem Umkristallisieren aus Methylenchlorid-Petrolether bei 177 179° schmilzt.
d) Ein Gemisch von 7,5 g 3ß,11ß-Diacetoxy-androst-5-en-17-on und 150 mg p-Toluolsulfonsäure in 450 ml Benzol und 7,5 ml Ethylenglykol wird 16 Stunden am Wasserabschneider unter Rückfluss gekocht. Nach Abkühlen wird die Lösung mit Ethylacetat verdünnt und sofort mit 225 ml eiskalter gesättigter Natriumchloridlösung gewaschen. Die organische Phase wird nach Trocknen im Wasserstrahlvakuum eingedampft und das ölige 3ß,llß-Diacetoxy-17,17-ethylendioxy-androst-5-en für die nächste Stufe ohne Reinigung verwendet.
e) Zu einer gerührten Suspension von 2,35 g Lithiumaluminiumhydrid in 95 ml Tetrahydrofuran wird bei der Innentemperatur von 5 - 10° eine Lösung von 4,7 g 3ß,11ß-Diacetoxy-17,17-ethylendioxy-androst-5-en in 140 ml Tetrahydrofuran zugetropft, mit 9 ml Tetrahydrofuran nachgespült, und das Gemisch 12 Stunden unter Rückfluss gekocht. Das Reaktionsgemisch wird bei einer Innentemperatur von max. 5° durch vorsichtiges Zutropfen eines Gemisches von 9 ml Tetrahydrofuran und 14 ml Ethylacetat und anschliessend eines Gemisches von 9 ml Tetrahydrofuran und 14 ml Wasser zersetzt und nach Zugabe von 70 g wasserfreiem Natriumsulfat noch 30 Minuten ohne Kühlung gerührt. Feste Anteile werden durch Abnutschen über eine Kieselgurschicht (Nachwaschen mit Tetrahydrofuran) entfernt, und das Filtrat im Wasserstrahlvakuum eingeengt. Der amorphe Rückstand wird an einer 50-fachen Gewichtsmenge Kieselgel chromatographiert. Durch Elution mit einem Gemisch von Methylenchlorid-Aceton (93 : 7) und Abdampfen des Lösungsmittels wird einheitliches 17,17-Ethylendioxy-androst-5-en-3ß,11ß-diol erhalten, das nach einmaligem Umlösen aus Methylenchlorid-Ether bei 123 -125° schmilzt.
f) Eine Lösung von 16,8 g 17,17-Ethylendioxy-androst-5-en-3ß,11ß-diol in 102 ml Tetrahydrofuran wird mit 36,3 g Pyridinhydrobromidperbromid versetzt und 2 1/2 Stunden bei Zimmertemperatur gerührt. Das Gemisch wird mit 26,9 g Natriumjodid versetzt, weitere 30 Minuten gerührt, nacheinander mit einer Lösung von 36,3 g :Natriumthiosulfat in 50,4 ml Wasser und mit 100 ml Pyridin versetzt und nochmals 2 Stunden bei Zimmertemperatur gerührt. Das Reaktionsgemisch wird mit 100 ml Wasser verdünnt und im Wasserstrahlvakuum bei ca. 45° eingeengt. Der Rückstand wird in Ethylacetat aufgenommen und nacheinander mit gesättigter Kochsalzlösung, eiskalter verdünnter Salzsäure, eiskalter verdünnter Natronlauge und nochmals mit gesättigter Kochsalzlosung gewaschen und über Natriumsulfat getrocknet. Durch Abdestillieren der Lösungsmittel im Wasserstrahlvakuum resultiert ein amorpher Rückstand von rohem 16a-Brom-17,17-ethylendioxy-androst-5-en-3ß,11ß-diol. Das erhaltene Rohprodukt (13 g) wird in 143 ml Dimethylsulfoxid gelöst, im Laufe von 30 Minuten bei 45° unter Rühren mit einem Gemisch von 7 g Kalium-tert-butoxid in 13 ml Dimethylsulfoxid versetzt und 20 Stunden bei 50° (Badtemperatur) gerührt. Das Gemisch wird auf die Raumtemperatur abgekühlt, mit ca. 1300 ml einer gesättigten Ammoniumchloridlösung verdünnt und in Ethylacetat aufgenommen; die organische Phase wird dreimal mit einer gesättigten Kochsalzlösung gewaschen und über Natriumsulfat getrocknet. Durch Abdestillieren der Lösungsmittel im Wasserstrahlvakuum wird amorphes 17,17-Ethylendioxy-androsta-5,15-dien-3ß,llß-diol erhalten, dessen Reinheit für die Weiterverarbeitung genügt.
g) Eine Lösung von 800 mg 17,17-Ethylendioxy-androsta-5,15-dien-3ß,11ß-diol in 40 ml Aceton wird mit 4 ml einer Lösung von 100 mg p-Toluolsulfonsäure in 10 ml Wasser versetzt und 6 Stunden bei Zimmertemperatur gerührt. Nach dem Verdünnen mit 40 ml Wasser wird das Aceton am Wasserstrahlvakuum abdestilliert, der ölige Rückstand in Chloroform aufgenommen und 1x mit eiskalter gesättigter Natriumhydrogencarbonatlösung gewaschen. Nach Abdampfen der organischen Lösungsmittel wird amorphes 3β,11β-Dihydroxy-androsta-5,16-dien-17-on erhalten, das ohne weitere Reinigung für die nächste Stufe verwendet werden kann.
h) Dimethylsulfoxid (64 ml) wird unter Stickstoffatmosphäre mit 1,52 g 55 bis 60-%-igem Natriumhydrid (als Mineralöl-Suspension) und 7,57 g Trimethylsulfoxoniumjodid versetzt und zuerst 30 Minuten bei Zimmertemperatur und anschliessend noch weitere 30 Minuten bei einer Aussentemperatur von 34 - 40° gerührt. Dem auf Zimmertemperatur abgekühlten Gemisch wird 8 g 3ß,11ß-Dihydroxy-androsta-5,15-dien-17-on zugegeben und mit 26 ml Dimethylsulfoxid nachgespült. Das Reaktionsgemisch wird 3 Stunden bei Zimmertemperatur gerührt, auf 1 Liter eiskalte gesättigte Kochsalzlösung gegossen mit wenig Methylalkohol und Wasser nachgespült, mit verdünnter Salzsäure angesäuert und 30 Minuten gerührt. Das abgeschiedene Öl wird in Ethylacetat aufgenommen und die organische Phase hintereinander mit gesättigter Kochsalzlösung, eiskalter verdünnter Natronlauge und wieder mit gesättigter Kochsalzlösung gewaschen. Nach dem Trocknen werden die Lösungsmittel im Wasserstrahlvakuum abgedampft und das resultierende amorphe 3ß,11ß-Dihydroxy-15ß,16ß-methylen-androst-5-en-17-on ohne Reinigung der anschliessenden Acetylierung unterworfen.

i) Eine Lösung von 7,9 g 3ß,11ß-Dihydroxy-15ß,16ß-methylen-androst-5-en-17-on in 39,5 ml Pyridin und 39,5 ml Essigsäureanhydrid wird 5 Stunden bei Zimmertemperatur stehen gelassen, mit 800 ml Eiswasser verdünnt und nach einstündigem Stehen mit Ethylacetat extrahiert. Die organische Phase wird nacheinander mit gesättigter Kochsalzlösung, eiskalter verdünnter Salzsäure, eiskalter verdünnter Natronlauge und nochmals mit gesättigter Kochsalzlösung gewaschen, getrocknet und im Wasserstrahlvakuum eingeengt. Durch Chromatographie des Rohproduktes an einer 30-fachen Gewichtsmenge Kieselgel und Elution mit einem Gemisch von Methylenchlorid-Aceton (98 : 2) wird 3ß-Acetoxy-11ß-hydroxy-15ß,16ß-methyten-androst-5-en-17-on erhalten, das nach einmaligem Umlösen aus Methylenchlorid-Ether-Petrolether bei 209 - 211° schmilzt.
j) Eine Lösung von 1,75 g 3ß-Acetoxy-11ß-hydroxy-15ß,16ß-methylen-androst-5-en-17-on in 10,5 ml Dimethylformamid und 3,5 ml y-Collidin wird mit 2,6 ml einer Lösung von 5 Gewichts-% Schwefeldioxid in Methansulfonsäurechlorid versetzt und 20 Minuten gerührt, wobei man die Innentemperatur auf etwa 45° steigen lässt. Das Gemisch samt dem ausgefällten Niederschlag wird auf 17,5 ml Eiswasser unter Rühren gegossen und noch weitere 10 Minuten gerührt. Das ausgeschiedene Öl wird in Ethylacetat aufgenommen und nacheinander mit gesättigter Kochsalzlösung, eiskalter verdünnter Salzsäure, eiskalter verdünnter Natronlauge und nochmals mit gesättigter Kochsalzlösung gewaschen. Nach dem Abdampfen der Lösungsmittel resultiert dünnschichtchromatographisch einheitliches 3ß-Acetoxy-15ß,16ß-methylen-androsta-5,9(11)-dien-17-on, das ohne Reinigung weiterverarbeitet wird.
k) Eine Lösung von 5,2 g 3ß-Acetoxy-15ß,16ß-methylen-androsta-5,9(11)-dien-17-on in 127,5 ml Tetrahydrofuran wird unter Eiswasserkühlung mit 1,78 g Lithiumdraht (Stücke von ca. 5 mm Länge) und danach innert 10 Minuten mit einer Lösung von 12,75 ml cyclischem Ethylenacetal des β-Chlorpropionaldehyds in 12,75 ml Tetrahydrofuran tropfenweise versetzt und anschliessend eine Stunde unter Eiskühlung und 16 Stunden bei Zimmertemperatur gerührt. Das Reaktionsgemisch wird mit 330 ml Ethylacetat versetzt und 45 Minuten gerührt, mit weiterem Ethylacetat verdünnt, nacheinander mit gesättigter Kochsalzlösung, eiskalter verdünnter Salzsäure, eiskalter verdünnter Natronlauge und nochmals mit gesättigter Kochsalzlösung gewaschen, getrocknet und im Wasserstrahlvakuum eingeengt. Das ölige Rohprodukt wird in einem Gemisch von Toluol-Ethylacetat (90 : 10) gelöst und durch eine 10-fache Gewichtsmenge Kieselgel filtriert. Das Filtrat liefert nach Abdampfen der Lösungsmittel 4,84 g amorphe Substanz. Diese wird in 363 ml Chloroform gelöst, mit 242 g saurem Aluminiumoxid (Aktivitäts-Stufe 1) versetzt und 2 1/2 Stunden bei Rückfluss Temperatur gerührt, mit weiteren 363 ml Chloroform verdünnt und noch weitere 5 Minuten gerührt und abgekühlt. Das Gemisch wird über Kieselgur genutscht, der Filterkuchen mit Chloroform nachgewaschen, und das Filtrat im Wasserstrahlvakuum eingeengt. Das erhaltene rohe 21-Carbaldehyd (4 g) wird in 20 ml Methylenchlorid und 80 ml Aceton gelöst, bei 5° während 5 Minuten mit 8 ml einer 8-N-Chrom(VI)-schwefelsäure-Lösung versetzt und 45 Minuten unter Eiskühlung gerührt. Das Gemisch wird mit 80 ml eiskaltem Wasser verdünnt, 10 Minuten ohne Kühlung gerührt und mit Methylenchlorid extrahiert. Die organische Phase wird mit eiskalter gesättigter Natriumhydrogencarbonatlösung gewaschen und getrocknet. Durch Abdestillieren der Lösungsmittel im Wasserstrahlvakuum erhält man kristallines Rohprodukt, welches in einer Lösung in Methylenchlorid durch eine 5-fache Gewichtsmenge neutrales Aluminiumoxid filtriert wird. Durch Abdestillieren des Lösungsmittels aus der Hauptfraktion werden Kristalle erhalten, die nach einmaligem Umlösen aus Methylenchlorid-Ether das 3ß-Acetoxy-15ß,16ß-methylen-20-spiroxa-5,9(11)-dien-21-on vom Smp. 241 - 243° liefert.
1) Zu einer Suspension von 1,9 g 3ß-Acetoxy-15ß,16ß-methylen-20-spiroxa-5,9(11)-dien-21-on in 26,6 ml Chloroforn und 190 ml Methylalkohol werden 19 ml einer 1-N Natriumhydroxidlösung zugegeben. Das Gemisch wird 1 Stunde bei Zimmertemperatur gerührt, mit 190 ml Wasser verdünnt und mit je einer Portion von Chloroform und eines Gemisches von Chloroform-Methanol (90 : 10) extrahiert. Die vereinigten organischen Phasen werden nach Trocknen im Wasserstrahlvakuum eingeengt und das kristalline Rohprodukt wird einmal aus Methylenchlorid-Ether-Petrolether umkristallisiert. Das erhaltene 3ß-Hydroxy-15ß,16ß-methylen-20-spiroxa-5,9(11)-dien-21-on schmilzt bei 244- 246°.
m) Aus einer Suspension von 400 mg 3ß-Hydroxy-15ß,16ß-methylen-20-spiroxa-5,9(11)-dien-21-on in 20 ml Toluol und 3 ml Cyclohexanon werden bei Normaldruck 4 ml Lösungsmittel abdestilliert. Es wird auf eine Innentemperatur von ca. 80° abgekühlt 480 mg Aluminiumisopropylat zugesetzt und zwei Stunden unter Rückfluss gerührt. Die Lösung wird auf Zimmertemperatur abgekühlt, mit einer Lösung von 0,4 ml Essigsäure in 0,8 ml Toluol versetzt und viermal mit je 5 ml Wasser im Wasserstrahlvakuum zur Trockne abgedampft. Der ölige Rückstand wird in Chloroform aufgenommen, nacheinander mit eiskalter verdünnter Salzsäure, Wasser, eiskalter Natronlauge und nochmals Wasser gewaschen, die organische Phase getrocknet und im Wasserstrahlvakuum abgedampft. Das amorphe Rohprodukt wird auf eine 50-fache Gewichtsmenge Kieselgel aufgetragen und mit einem Gemisch von Methylenchlorid-Aceton (98 : 2) chromatographiert. Das erhaltene 15ß,16ß-Methylen-20-spiroxa-4,9(11)-dien-3,21-dion schmilzt nach einmaligem Umlösen aus Methylenchlorid- Ether-Petrolether bei 172 -174°.
n) Eine Lösung von 3,27 g 15ß,16ß-Methylen-20-spiroxa-4,9(11)-dien-3,21-dion in 16,35 ml Dioxan und 6,54 ml Orthoameisensäuretrimethylester wird mit 0,654 ml einer Lösung von 900 mg p-Toluolsulfonsäure in 10 ml Dioxan und 2 ml Ethylalkohol vermischt, 4 Stunden bei Raumtemperatur gerührt, unter Rühren in 430 ml einer eiskalten 0,2-N-Natriumhydroxidlösung gegossen und 15 Minuten intensiv gerührt. Der Niederschlag wird abgenutscht, mit Wasser gewaschen und auf der Nutsche getrocknet. Das erhaltene rohe 3-Ethoxy-15ß,16ß- methylen-20-spiroxa-3,5,9(11)-trien-21-on wird in 105 ml Aceton gelöst und nacheinander mit einer Lösung von 1,13 g Natriumacetat (Trihydrat) in 8,84 ml Wasser und, unter Kühlung auf -5°, mit 1,55 g N-Bromacetamid und 1,13 ml Essigsäure behandelt. Das Gemisch wird 30 Minuten bei einer Innentemperatur von ca. -3° und nachher noch 15 Minuten ohne Kühlung gerührt, mit einer Lösung von 0,88 g Kaliumjodid in 17,7 ml Wasser und einer Lösung von 5,58 g Natriumthiosulfat in 17,7 ml Wasser nacheinander versetzt, noch weitere 5 Minuten gerührt und mit 88 ml Wasser verdünnt. Das Gemisch wird mit Chloroform extrahiert und die organische Phase mit eiskalter gesättigter Natriumhydrogencarbonatlösung gewaschen. Durch Trocknen und Einengen der organischen Phase wird der amorphe Rückstand erhalten, der in 78 ml Dimethylformamid gelöst, mit 3,89 g Lithiumcarbonat und 3,89 g Lithiumbromid versetzt und 3 Stunden bei 100° gerührt wird. Das abgekühlte Gemisch wird unter Rühren auf 750 ml Eiswasser gegossen, und der Niederschlag abgenutscht und mit Wasser gewaschen. Der Filterkuchen wird in Chloroform gelöst, mit Natriumsulfat getrocknet und im Wasserstrahlvakuum zur Trockne abgedampft. Die Lösung des erhaltenen Rückstands in Methylenchlorid wird durch eine Säule von neutralem Aluminiumoxid (Aktivität 11) filtiert und mit weiteren Anteilen desselben Lösungsmittels eluiert. Die Eluate werden eingeengt und das gewünschte 15ß,16ß-Methylen-20-spiroxa-4,6,9(11)-trien-3,21-dion in amorpher Form durch Zugabe von Ether gefällt. Das Produkt ist gemäss Dünnschichtchromatographie einheitlich und zur Weiterverarbeitung geeignet.

### Beispiel 8

Ein Gemisch von 2,0 g 9a,11a-Epoxy-6ß,7ß-methylen-20-spirox-4-en-3,21-dion, 40 ml Methylalkohol und 2,45 ml 2-N wässriger Lösung von Kaliumhydroxid wird 16 Stunden bei 60° gerührt. Die Reaktionslösung wird im Wasserstrahlvakuum eingedampft und durch dreimaliges Eindampfen mit absolutem Ethylalkohol vom restlichen Wasser befreit. Der Rückstand wird in heissem Methanol gelöst und mit Ethylacetat kristallinisch gefällt. Das resultierende Katium-9a,11a-epoxy-17ß-hydroxy-6ß,7ß-methyten-3-oxo-17a-pregn-4-en-21-carboxylat wird im Hochvakuum bei 80° getrocknet. Analyse: berechnet 8,83 % K; gefunden 8,76 % K.

Dasselbe Kaliumsalz wird unter analoger Arbeitsweise ausgehend von einer äquivalenten Menge 9a,11a-Epoxy-17ß-hydroxy-6ß,7ß-methylen-3-oxo-17a-pregn-4-en-21-carbonsäure-methylester erhalten.

In analoger Weise kann auch 9a,11a-Epoxy-6ß,7ß;15ß,16ß-bis-methylen-ZO-spirox-4-en-3,21-dion bzw. 9a,11a-Epoxy-17ß-hydroxy-6ß,7ß;15ß,16ß-bis-methylen-3-oxo-17a-pregn-4-en-21-carbonsäure-methylester zum Kaliumsalz der letztgenannten Säure umgewandelt werden.

### Beispiel 9

Eine Suspension von 1,8 g 9α,11α-Epoxy-6ß.7ß-methylen-20-spirox-4.-en-3,21-dion in 18 ml Methylalkohol wird mit 1,5 ml 4-N methanolischer Kaliumhydroxid-Lösung versetzt und 1 Stunde am Rückfluss gekocht. Bei Normaldruck werden ca. 12 ml des Lösungsmittels abdestilliert, 18 ml Ethylacetat zugegeben, und das Gemisch wird im Wasserstrahlvakuum bis auf ca. 12 ml eingeengt. Nach erneuter Zugabe von 18 ml Ethylacetat wird der gebildete kristalline Niederschlag noch 10 Minuten verrührt, abgenutscht, mit Ethylacetat und Ether gewaschen und auf der Nutsche getrocknet. Das gelbliche Pulver wird in 9 ml Dimethylformamid gelöst, mit 0,9 ml Methyljodid versetzt und 40 Stunden unter Verschluss bei Zimmertemperatur gerührt. Das Gemisch wird mit Eiswasser verdünnt und 15 Minuten gerührt. Der ausgefallene Niederschlag wird abgenutscht, mit Wasser ausgewaschen, in Chloroform gelöst und die organische Phase nach Trocknen im Wasserstrahlvakuum eingedampft. Das amorphe Rohprodukt wird auf eine 30-fache Gewichtsmenge Kieselgel aufgetragen und mit einem Gemisch von Methylenchlorid-Aceton (95 : 5) eluiert. Die vereinigten einheitlichen Fraktionen ergeben nach einmaligem Umlösen aus Methylenchlorid-Ether 9α,11α-Epoxy-17ß-hydroxy-6ß,7ß-methylen-3-oxo-17α-pregn-4-en-21-carbonsäure-methylester, Smp.189 -191 °.

In analoger Weise kann man anstelle von Methyljodid mit einer äquivalenten Menge Dimethylsulfat umsetzen.

### Beispiel 10

Eine Lösung von 1,3 g 9a, 11a-Epoxy-6ß, 7ß-methylen-20-spirox-4-en-3,21-dion und 1,3 g DDQ (2,3-Dichlor-5,6-dicyanbenzochinon) in 26 ml Dioxan wird 15 Stunden bei 100° gerührt. Das dunkle Reaktionsgemisch wird dreimal mit Toluol im Wasserstrahlvakuum eingedampft, der Rückstand in Methylenchlorid gelöst und durch eine 10-fache Gewichtsmenge Aluminiumoxid (neutral) filtriert. Die erhaltenen Kristalle ergeben nach einmaligem Umlösen aus Methylenchlorid-Ether das gewünschte 9a,11a-Epoxy-6ß,7ß-methyien-20-spiroxa-1,4-dien-3,21-dion, Smp. 295 - 296°.

### Beispiel 11

Ein Gemisch von 1,3 g 9a,11a-Epoxy-7a-methoxycarbonyl-20-spirox-4-en-3,21-dion, 17 ml Methylalkohol und 1,41 ml 2-N wässriger Lösung von Kaliumhydroxid wird 16 Stunden bei Zimmertemperatur und anschliessend 20 Minuten bei 60° gerührt. Die Reaktionslösung wird im Wasserstrahlvakuum eingedampft und durch dreimaliges Eindampfen mit absolutem Ethylalkohol vom restlichen Wasser befreit. Der kristalline Rückstand wird mit 40 ml Ethylacetat verrührt, abgenutscht und am Filter mit Ethylacetat und Ether ausgewaschen. Das resultierende flockige Kalium-9α,11α-epoxy-17ß-hydroxy-7α-methoxycarbonyl-3-oxo-17α-pregn-4-en-21-carboxylat wird im Hochvakuum bei 80° getrocknet. Analyse: berechnet 8,31 % K; gefunden 8,02 % K. Dasselbe Salz wird auch durch analoges Umsetzen einer äquivalenten Menge 9a,11a-Epoxy-17ß-hydroxy-3-oxo-17ß-pregn-4-en-7a,21-dicarbonsäure-dimethylester erhalten.

### Beispiel 12

Ein Gemisch von 1,25 g 9a.11a-Epoxy-7a-isopropoxycarbonyl-20-spirox-4-en-3,21-dion, 15,6 ml Methylalkohol und 1,27 ml 2-N wässriger Lösung von Kaliumhydroxid wird 16 Stunden bei Zimmertemperatur und anschliessend 20 Minuten unter Rückfluss gerührt. Die Reaktionslösung wird im Wasserstrahlvakuum eingedampft und durch dreimaliges Eindampfen mit absolutem Ethylalkohol vom restlichen Wasser befreit. Der kristalline Rückstand wird mit 40 ml Ethylacetat verrührt, abgenutscht und am Filter mit Ethylacetat und Ether ausgewaschen. Das resultierende flockige Kalium-9a,lla-epoxy-17ß-hydroxy-7a-isopropoxycarbonyl-3-oxo-17a-pregn-4-en-21-carboxylat wird im Hochvakuum bei 80° getrocknet. Analyse: berechnet 7,66 % K; gefunden 7,15 % K.

In analoger Weise wird ausgehend von einer äquivalenten Menge 9a,11a-Epoxy-7a-ethoxycarbonyl-20- spirox-4-en-3,21-dion oder 9α,11α-Epoxy-17ß-hydroxy-3-oxo-17ß-pregn-4-en-7α,21-dicarbonsäure-7-ethylester-21-methylester das Kaliumsalz der 9α,11α-Epoxy-7α-ethoxycarbonyl-17ß-hydroxy-3-oxo-17ß-pregn-4-en-21-carbonsäure erhalten, dessen Analyse (Kaliumgehalt) der Theorie entspricht.

### Beispiel 13

Ein Gemisch von 5,7 g Trimethylsulfoxoniumjodid in 19 ml Dimethylsulfoxid wird unter strengem Feuchtigkeitsausschluss mit 0,82 g einer 72-%-iger (G/V) Suspension von Natriumhydrid in Mineralöl 1 Stunde bei Zimmertemperatur gerührt, mit 1,46 g 9a,11a-Epoxy-20-spiroxa-4,6-dien-3,21-dion [vgl. J. Med. Chem., 6, (1963) 732 - 735] versetzt, mit 1,46 ml Dimethylsulfoxid nachgespült und während 3,5 Stunden bei Zimmertemperatur weitergerührt. Das Reaktionsgemisch wird mit 146 ml Eiswasser verdünnt, mit verdünnter Salzsäure angesauert und mit Ethylacetat extrahiert. Die organische Phase wird nacheinander mit gesättigter Kochsalz-Lösung, eiskalter verdünnter Natronlauge und wieder mit gesättigter Kochsalz-Lösung gewaschen, über Natriumsulfat getrocknet und im Wasserstrahlvakuum eingedampft. Das erhaltene amorphe Rohprodukt wird an einer 100-fachen Gewichtsmenge Kieselgel chromatographiert. Durch Eluieren mit einem Gemisch von Toluol-Aceton (95 : 5) und Eindampfen resultiert das 9a,11a-Epoxy-6a,7a-methylen-20-spirox-4-en-3,21-dion, Smp. 262 - 264° (nach einmaligem Umlösen aus Methylenchlorid/Ether).

### Beispiel 14

Ein Gemisch von 10 g 9a,11a-Epoxy-7a-methoxycarbonyl-20 spirox-4-en-3,21-dion, 100 ml Methylalkohol und 7,5 ml 4-N methanolischer Lösung von Kaliumhydroxid wird während 1 Stunde am Rückfluss gekocht. Aus dem Reaktionsgemisch werden bei Normaldruck ca. 80 ml abdestilliert, 60 ml Ethylacetat zugesetzt und die Suspension im Wasserstrahlvakuum auf ca. 20 ml eingeengt. Nach erneutem Verdünnen mit 60 ml Ethylacetat wird das Gemisch 30 Minuten bei Zimmertemperatur stehen gelassen, worauf der Niederschlag abgenutscht und zweimal mit Ether ausgewaschen wird. Der pulverige Rückstand wird in 46 ml Dimethylformamid suspendiert und nach der Zugabe von 4,6 ml Methyljodid während 40 Stunden unter Verschluss bei Zimmertemperatur gerührt. Nach dem Verdünnen mit 300 ml Eiswasser wird der Niederschlag abgetrennt, mit Wasser gewaschen, in Ethylacetat gelöst und die Lösung nach Trocknen im Wasserstrahlvakuum eingedampft. Das erhaltene Rohprodukt wird an 30-facher Menge Kieselgel chromatographiert. Die mit einem Gemisch von Methylenchlorid-Aceton (92 : 8) eluierten einheitlichen Fraktionen liefern nach dem Bespritzen mit Ether und einmaligem Fällen des gebildeten Niederschlags aus Methylenchlorid mit Ether den gewünschten 9a,11a-Epoxy-17ß-hydroxy-3-oxo-17a-pregn-4-en-7a,21-dicarbonsäure-dimethylester in amorpher Form.

In analoger Weise kann man 9a,11a-Epoxy-7a-methoxy-15ß,16ß-methylen-20-spirox-4-en-3,21-dion zum 9a,11 a-Epoxy-17ß-hydroxy-15ß,16ß-methylen-3-oxo-17a-pregn-4-en-7a,21-dicarbonsäure-dimeₜhylester umwandeln.

### Beispiel 15

Tabletten, enthaltend ca. 20 mg Wirkstoff, z. B. 9a,11a-Epoxy-7a-methoxycarbonyl-20-spirox-4-en-3,21-dion oder 9a,11a-Epoxy-6ß,7ß-methylen-20-spirox-4-en-3,21-dion, werden wie folgt hergestellt:

### Zusammensetzung für 1000 Tabletten

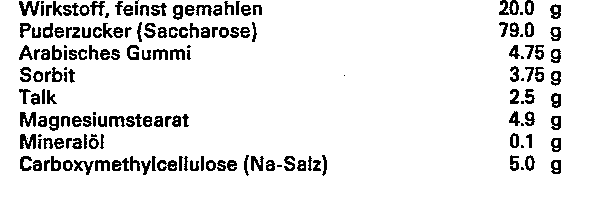

### Herstellung:

Der Wirkstoff wird mit dem Puderzucker und dem arabischen Gummi vermischt, gesiebt und mittels einer etwa 35-prozentigen wässrigen Sorbit-Lösung granuliert. Das Granulat wird durch ein Sieb getrieben, getrocknet, nochmals gesiebt und mit den übrigen Hilfsstoffen (Talk, Magnesiumstearat, Mineralöl und Carboxymethylcellulose-Natriumsalz) innig vermischt. Die Mischung wird in üblicher Weise zu Tabletten von 120 mg verpresst.

### Beissiel 16

Gelatinekapseln enthaltend ca. 25 mg Wirkstoff, z. B. 9α,11α-Epoxy-6ß,7ß-methylen-20-spirox-4-en-3,21-dion, werden folgendermassen hergestellt:

### Zusammensetzung für 1000 Kapseln

### Wirkstoff, feinst gemahlen 25 g

### Lactose, feinst gemahlen 25 g

Der Wirkstoff und die Lactose werden innig vermischt, verrieben und gesiebt, und das erhaltene Pulver in Portionen zu je 50 mg in Gelatinekapseln abgefüllt.

### Beispiel 17:

Tabletten, enthaltend ca. 25 mg Komponente A und ca. 25 mg Komponente B, werden folgendermassen hergestellt:

### Zusammensetzuna einer Tablette:

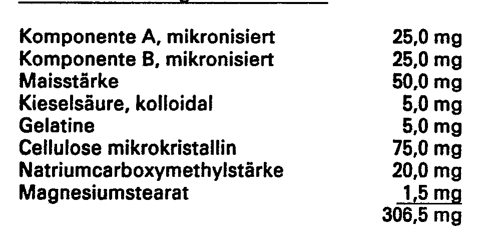

### Herstellung von 100 000 Tabletten

2,5 kg Komponente A, mikronisiert, 2,5 kg Komponente B, mikronisiert, und 5,0 kg Maisstärke werden mit 0,5 kg kolloidaler Kieselsäure gemischt und mit einer Lösung von 0,5 kg Gelatine in 5,0 kg destilliertem Wasser (30°C) zu einer feuchten Masse verarbeitet. Diese wird durch ein Sieb von 3 mm Maschenweite getrieben und bei 45°C getrocknet (Wirbelschichttrockner). Das trockene Granulat wird durch ein Sieb von 0,8 Maschenweite gedrückt, mit einer im voraus gesiebten Mischung von 7,5 kg mirkokristalliner Cellulose und 2,0 kg Natriumcarboxymethylstärke sowie 0,15 kg Magnesiumstearat gemischt und zu Tabletten von 306,5 mg Gewicht verpresst.

Als Komponente A wird z. B.:
9α,11α-Epoxy-7α(methoxycarbonyl oder isopropoxycarbonyl)-20-spirox-4-en-3,21-dion, 9α,11α-Epoxy-6ß,7ß- methylen-20-spirox-4-en-3,21-dion oder
9a,11a-Epoxy-6ß,7ß;15ß,16ß-bis-methylen-20-spirox-4-en-3,21-dion,
als Komponente B:
6-Chlor-7-sulfamyl-3,4-dihydro-1,2,4-benzothiadiazin-1,1-dioxid verwendet.

In analoger Weise kann man auch in äquivalenten Mengen die folgenden Wirkstoffe einsetzen:
Als Komponente A:
Das Kalium- oder Natriumsalz der
9α,11α-Epoxy-17ß-hydroxy-6ß,7ß-methylen-3-oxo-17α-pregn-4-en-21-carbonsäure (50 mg) oder 9a,11a-Epoxy-17ß-hydroxy-7a-methoxycarbonyt-3-oxo-17a-pregn-4-en-21-carbonsäure (30 mg);
als Komponente B:
2-Chlor-5-(3-hydroxy-1-oxo-isoindolyl-3)-benzolsulfonamid oder
4-(2-Methylenbutyryl)-2,3-dichlorphenoxyessigsäure (je 50 mg),
6-Chlor-7-sulfamyl-3,4-dihydro-1,2,4-benzothiadiazin-1,1-dioxid (25 mg),
2-Phenoxy-3-butylamino-5-carboxybenzolsulfonamid (0,5 mg),
(1-0xo-2-methyl)-2-phenyl)-6,7-dichlor-indanyl-5-oxy)-esslgsäure (als Razemat 20 mg, als die laevo-Form 10 mg) oder
3-Cyclopentylmethyl-6-chlor-7-sulfamyl-3,4-dihydro-1,2,4-benzothiadiazin-1,1-dioxid (0,5 mg).

## Patentansprüche (Patentansprüche für folgende(n) Vertragsstaat(en) : BE, CH, DE, FR, GB, IT, LI, LU, NL, SE)

1. Eine Steroidverbindung der Formel 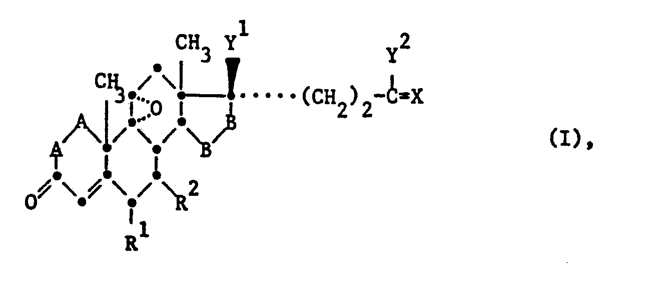 worin
-A-A- die Gruppe -CH₂₋CH₂- oder -CH = CH-,
R' Wasserstoff und
R² ein a-orientiertes Niederalkoxycarbonyl, oder
R' und R² zusammen ein a- oder β-orientierter Methylenrest,
-B-B- die Gruppe -CH₂-CH₂- oder eine a- oder β-orientierte Gruppe 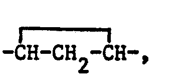
X zwei Wasserstoffatome oder Oxo,
Y¹ und Y2 zusammen die Sauerstoffbrücke -0- oder
Y¹ Hydroxyl und
Y² Hydroxyl, Niederalkoxy oder, falls X gleich H₂ ist, auch Niederalkanoyloxy ist, sowie Salze von Verbindungen, worin X Oxo und Y² Hydroxyl ist, wobei mit "nieder" bezeichnete organische Reste höchstens 7 Kohlenstoffatome enthalten.

2. Eine Verbindung gemäss Anspruch 1, worin R² Methoxycarbonyl, Ethoxycarbonyl oder Isopropoxycarbonyl bedeutet.

3. Eine Verbindung gemäss Anspruch 1, worin der durch die gemeinsame Bedeutung von R¹ und R² dargestellte Methylenrest und/oder die durch das Symbol -B-B- dargestellte Gruppe 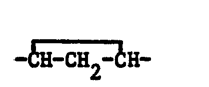 β-orientiert ist.

4. Eine Verbindung gemäss einem der Ansprüche 1 - 3, worin X für Oxo steht und Y¹ und Y² zusammen die Sauerstoffbrücke -0- bedeuten.

5. Ein Alkalimetallsalz einer Verbindung gemäss einem der Ansprüche 1 - 3, worin X für Oxo und Y¹ und Y² jedes für Hydroxyl stehen.

6. Eine Verbindung gemäss Anspruch 1, die 9a,11a-Epoxy-7a-methoxycarbonyl-20-spirox-4-en-3,21-dion ist.

7. Eine Verbindung gemäss Anspruch 1, die 9α,11α-Epoxy-6ß,7ß-methylen-20-spirox-4-en-3,21-dion ist.

8. Eine Verbindung gemäss einem der Ansprüche 1 - 7 zur Verwendung als Aldosteron-antagonisierender Wirkstoff.

9. Verfahren zur Herstellung der im Anspruch 1 definierten Steroidverbindungen der Formel I einschliesslich Salze von Verbindungen, worin X Oxo und Y² Hydroxyl ist, dadurch gekennzeichnet, dass man
a) eine Verbindung der Formel 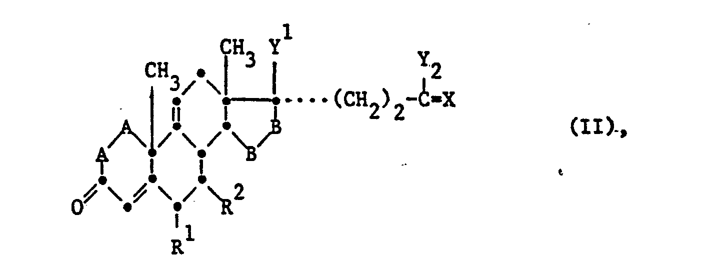 worin A-A, B-B, R¹, R², X, Y¹ und Y² die obengenannten Bedeutungen haben, mit einer Peroxysäure behandelt, oder
b) eine Verbindung der Formel 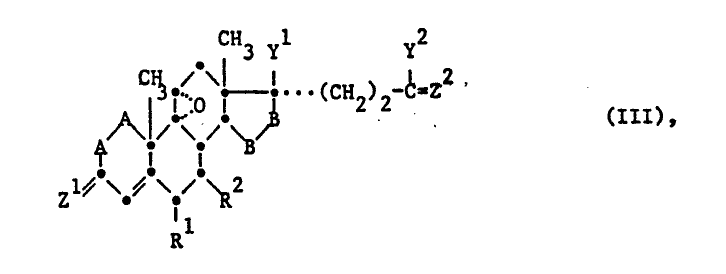 worin A-A, B-B, R¹, R², Y¹ und Y² die obengenannten Bedeutungen haben und mindestens eines der Symbole Z¹ und Z² für Hydroxyl zusammen mit Wasserstoff steht und das andere dieselbe Bedeutung hat oder für Oxo steht oder, als das Symbol 2², auch für zwei Wasserstoffatome stehen kann, mit einem Oxidationsmittel behandelt, oder
c) zur Herstellung einer Verbindung, worin Y¹ Hydroxyl und die übrigen Symbole die obengenannten Bedeutungen haben, eine Verbindung der Formel 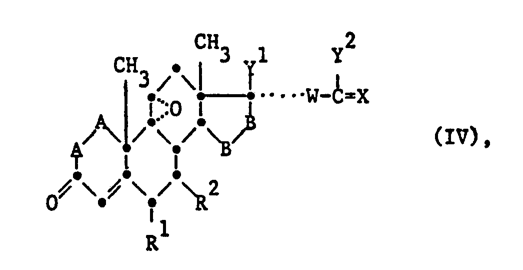 worin A-A, B-B, R¹, R², X und Y² die oben angegebenen Bedeutungen haben, Y¹ Hydroxyl und W eine Gruppe -CH=CH- oder -C≡C- bedeutet, zur Sättigung der Mehrfachbindung in der Seitenkette hydriert, oder
d) zur Herstellung einer Verbindung der Formel I, worin Y¹ und Y² zusammen die Sauerstoffbrücke -0- bedeuten und die übrigen Symbole die obengenannten Bedeutungen haben, eine Verbindung der Formel 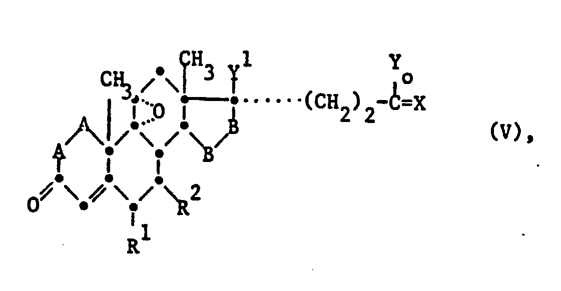 worin A-A, B-B, R¹, R² und X die obengenannten Bedeutungen haben, Y¹ Hydroxyl und Yₒ eine Abgangsgruppe bedeutet, unter Abspaltung der Gruppe Yₒ cyclisiert, oder e) zur Herstellung einer Verbindung der Formel I, worin R¹ Wasserstoff und R² eine Niederalkoxycarbonylgruppe bedeutet und die übrigen Symbole die obengenannten Bedeutungen haben, eine Verbindung der Formel 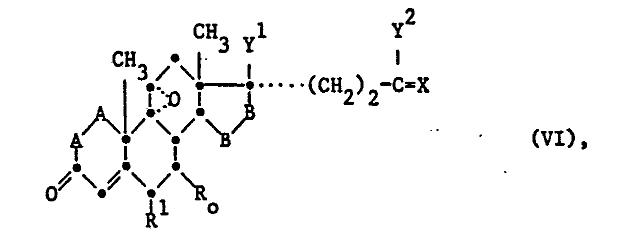 worin A-A, B-B, X, Y¹ und Y² die obengenannten Bedeutungen haben, R¹ Wasserstoff und Rₒ freies Carboxyl bedeutet, oder ein reaktionsfähiges Derivat oder Salz einer solchen Verbindung, in Ester umwandelt, oder
f) zur Herstellung einer Verbindung, worin R¹ und R² zusammen eine Methylenbrücke darstellen und die übrigen Symbole die obengenannten Bedeutungen haben, an eine Verbindung der Formel 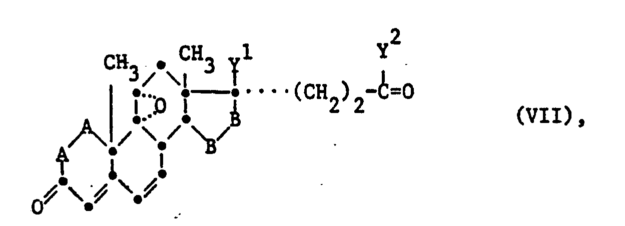 worin A-A, B-B, Y¹ und Y² die obengenännten Bedeutungen haben, die Methylengruppe anlagert und, wenn erwünscht,
g) eine erhaltene Verbindung der Formel 1, worin -A-A- für -CH₂-CH₂- steht, mit einem Dehydrierungsmittel zur Einführung der 1,2-Doppelbindung behandelt, und/oder
h) eine erhaltene Verbindung der Formel I, worin Y¹ und Y² je eine Hydroxylgruppe bedeutet, durch Abspaltung der Elemente von Wasser zur Verbindung der Formel I, worin Y¹ und Y² zusammen die Sauerstoffbrücke darstellen, cyclisiert, und/oder
i) eine erhaltene Verbindung der Formel I, worin X für zwei Wasserstoffatome steht, zu einer entsprechenden Verbindung, worin X für Oxo steht, oxidiert, und/oder
j) in einer erhaltenen Verbindung der Formel I, worin X zwei Wasserstoffatome bedeutet und je Y¹ und Y² eine freie Hydroxylgruppe darstellen, die endständige Hydroxylgruppe acyliert, und/oder
k) eine erhaltene Verbindung der Formel 1, worin X Oxo, Y¹ für Hydroxyl und Y² für Hydroxyl oder Niederalkoxy stehen oder beide zusammen die Sauerstoffbrücke -O- darstellen, in ein Salz der entsprechenden 17ß-Hydroxy-21-carbonsäure der Formel I, worin X für Oxo und je Y¹ und Y² für Hydroxyl stehen, überführt und/oder ein solches Salz zur freien Säure und/oder die freie Säure oder ein Salz davon zu einem Niederalkylester umwandelt.

10. Eine pharmazeutische Zusammensetzung enthaltend als Wirkstoff eine der in Ansprüchen 1 - 8 definierten Verbindungen, gewünschtenfalls zusammen mit einem im Bezug auf die Ausscheidung von Elektrolyten unspezifischen Diuretikum.

11. Verfahren zur Herstellung einer pharmazeutischen Zusammensetzung gemäss Anspruch 10, dadurch gekennzeichnet, dass man den dort definierten Wirkstoff (die Wirkstoffe) mit mindestens einem pharmazeutisch anwendbaren Trägermaterial auf nicht-chemischem Wege verarbeitet.

## Patentansprüche (Patentansprüche für folgende(n) Vertragsstaat(en) : AT)

1. Verfahren zur Herstellung von Steroidverbindungen der Formel 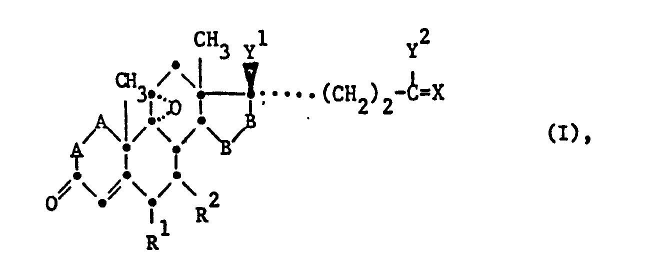 worin
-A-A- die Gruppe -CH₂-CH₂- oder -CH=CH-,
R¹ Wasserstoff und
R² ein a-orientiertes Niederalkoxycarbonyl, oder
R¹ und R² zusammen ein a- oder β-orientierter Methylenrest,
-B-B- die Gruppe -CH₂-CH₂ oder eine a- oder β-orientierte Gruppe 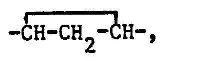
X zwei Wasserstoffatome oder Oxo,
Y¹ und Y² zusammen die Sauerstoffbrücke -0- oder
Y¹ Hydroxyl und
Y² Hydroxyl, Niederalkoxy oder, falls X gleich H₂ ist, auch Niederalkanoyloxy ist,
sowie von Salzen der Verbindungen, worin X Oxo und Y² Hydroxyl ist, wobei mit "nieder" bezeichnete organische Reste höchstens 7 Kohlenstoffatome aufweisen, dadurch gekennzeichent, dass man
a) eine Verbindung der Formel 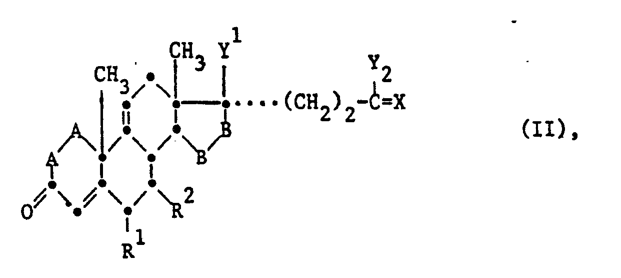 worin A-A, B-B, R¹, R², X, Y^{l} und Y² die obengenannten Bedeutungen haben, mit einer Peroxysäure behandelt, oder
b) eine Verbindung der Formel 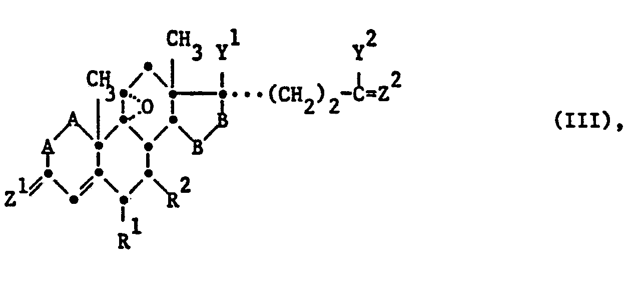 worin A-A, B-B, R¹, R², Y¹ und Y² die obengenannten Bedeutungen haben und mindestens eines der Symbole Z¹ und Z² für Hydroxyl zusammen mit Wasserstoff steht und das andere dieselbe Bedeutung hat oder für Oxo steht oder, als das Symbol Z², auch für zwei Wasserstoffatome stehen kann, mit einem Oxidationsmittel behandelt, oder
c) zur Herstellung einer Verbindung, worin Y¹ Hydroxyl und die übrigen Symbole die obengenannten Bedeutungen haben, eine Verbindung der Formel 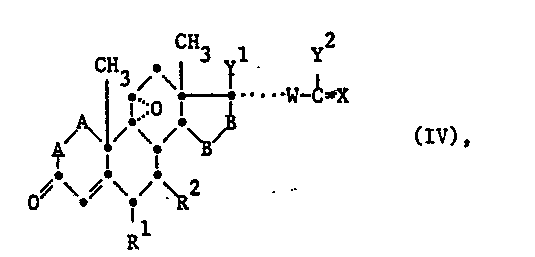 worin A-A, B-B, R¹, R², X und Y² die oben angegebenen Bedeutungen haben, Y¹ Hydroxyl und W eine Gruppe -CH=CH- oder -C≡C- bedeutet, zur Sättigung der Mehrfachbindung in der Seitenkette hydriert, oder
d) zur Herstellung einer Verbindung der Formel I, worin Y¹ und Y² zusammen die Sauerstoffbrücke -0- bedeuten und die übrigen Symbole die obengenannten Bedeutungen haben, eine Verbindung der Formel 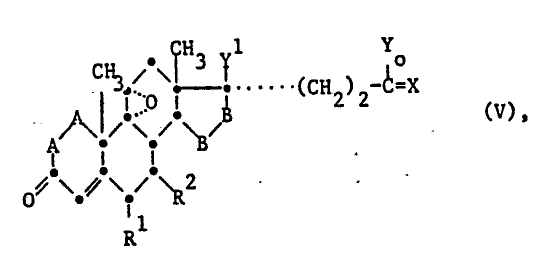 worin A-A, B-B, R¹, R² und X die obengenannten Bedeutungen haben, Y¹ Hydroxyl und Y eine Abgangsgruppe bedeutet unter Abspaltung der Gruppe Y cyclisiert, oder
e) zur Herstellung einer Verbindung der Formel I, worin R¹ Wasserstoff und R² eine Niederalkoxycarbonylgruppe bedeutet und die übrigen Symbole die obengenannten Bedeutungen haben, eine Verbindung der Formel 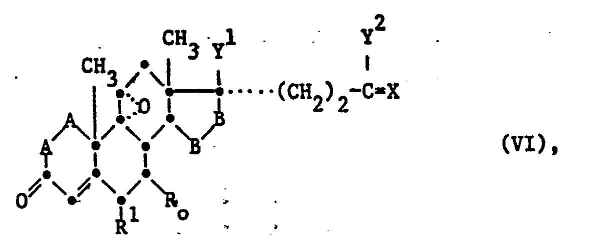 worin A-A, B-B, X, Y¹ und Y² die obengenannten Bedeutungen haben, R¹ Wasserstoff und Rₒ freies Carboxyl bedeutet, oder ein reaktionsfähiges Derivat oder Salz einer solchen Verbindung, in Ester umwandelt, oder
f) zur Herstellung einer Verbindung, worin R¹ und R² zusammen eine Methylenbrücke darstellen und die übrigen Symbole die obengenannten Bedeutungen haben, an eine Verbindung der Formel 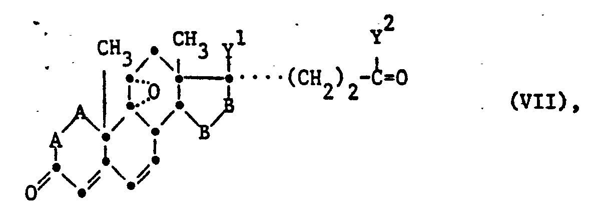 worin A-A, B-B, Y¹ und Y² die obengenannten Bedeutungen haben, die Methylengruppe anlagert und wenn erwünscht,
g) eine erhaltene Verbindung der Formel I, worin -A-A- für -CH₂-CH₂- steht, mit einem Dehydrierungsmittel zur Einführung der 1,2-Doppelbindung behandelt, und/oder
h) eine erhaltene Verbindung der Formel 1, worin Y¹ und Y² je eine Hydroxylgruppe bedeutet, durch Abspaltung der Elemente von Wasser zur Verbindung der Formel I, worin Y¹ und Y² zusammen die Sauerstoffbrücke darstellen, cyclisiert, und/oder
i) eine erhaltene Verbindung der Formel I, worin X für zwei Wasserstoffatome steht, zu einer entsprechenden Verbindung, worin X für Oxo steht, oxidiert, und/oder
j) in einer erhaltenen Verbindung der Formel I, worin X zwei Wasserstoffatome bedeutet und je Y¹ und Y² eine freie Hydroxylgruppe darstellen, die endständige Hydroxylgruppe acyliert, und/oder
k) eine erhaltene Verbindung der Formel 1, worin X für Oxo, Y¹ für Hydroxyl und Y² für Hydroxyl oder Niederalkoxy stehen oder beide zusammen die Sauerstoffbrücke -0- darstellen, in ein Salz der entsprechenden 17ß-Hydroxy-21-carbonsäure der Formel I, worin X für Oxo und je Y¹ und Y² für Hydroxyl stehen, überführt und/oder ein solches Salz zur freien Säure und/oder die freie Säure oder ein Salz davon zu einem Niederalkylester umwandelt.

2. Verfahren gemäss Anspruch 1, dadurch gekennzeichnet, dass man eine Verbindung der Formel worin R² Methoxycarbonyl, Ethoxycarbonyl oder Isopropoxycarbonyl bedeutet, herstellt.

3. Verfahren gemäss Anspruch 1, dadurch gekennzeichnet, dass man eine Verbindung der Formel 1 herstellt, worin R¹ und R² zusammen einen β-orientierten Methylenrest darstellen und/oder -B-B- die β-orientierte Gruppe 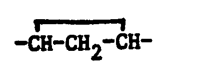 bedeutet.

4. Verfahren gemäss Anspruch 1, dadurch gekennzeichnet, dass man eine Verbindung der Formel herstellt, worin X für Oxo steht und Y¹ und Y² zusammen die Sauerstoffbrücke -0- bedeuten.

5. Verfahren gemäss einem der Ansprüche 1 - 3, dadurch gekennzeichnet, dass man ein Alkalimetallsalz einer Verbindung der Formel worin X für Oxo und Y¹ und Y² jedes für Hydroxyl stehen, herstellt. -

6. Verfahren gemäss Anspruch 4, dadurch gekennzeichnet, dass man ein Kaliumsalz herstellt.

7. Verfahren gemäss Anspruch 1, dadurch gekennzeichnet, dass man 9α,11α-Epoxy-7α-methoxycarbonyl-20- spirox-4-en-3,21-dion herstellt.

8. Verfahren gemäss Anspruch 1, dadurch gekennzeichnet, dass man 9a,11a-Epoxy-7a-isopropoxycarbonyl-20-spirox-4-en-3,21-dion herstellt.

9. Verfahren gemäss Anspruch 1, dadurch gekennzeichnet, dass man 9α,11α-Epoxy-6ß,7ß-methylen-20- spirox-4-en-3,21-dion herstellt.

10. Verfahren zur Herstellung einer pharmazeutischen Zusammensetzung enthaltend als Wirkstoff eine der in einem der Ansprüche 1 - 9 bezeichneten Verbindungen, gewünschtenfalls zusammen mit einem im Bezug auf die Ausscheidung von Elektrolyten unspezifischen Diuretikum, dadurch gekennzeichnet, dass man den Wirkstoff (die Wirkstoffe) mit mindestens einem pharmazeutisch anwendbaren Trägermaterial auf nicht-chemischem Wege verarbeitet.

11. Eine gemäss Anspruch 10 hergestellte pharmazeutische Zusammensetzung.

## Claims (Claims for the following Contracting State(s) : s BE, CH, DE, FR, GB, IT, LI, LU, NL, SE)

1. A steroid compound of the formula 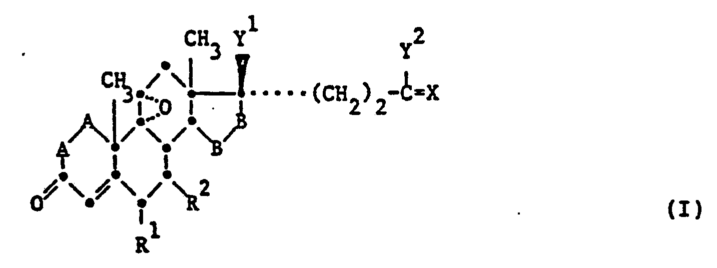 in which
-A-A- represents the group -CH₂-CH₂- or -CH =CH-,
R¹ represents hydrogen, and
R² represents an a-oriented lower alkoxycarbonyl radical, or
R¹ and R² together represent an a- or ß-oriented methylene radical,
-B-B- represents the group -CH₂-CH₂- or an a- or ß-oriented group 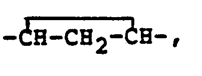
X represents two hydrogen atoms or oxo,
Y¹ and Y² together represent the oxygen bridge -0-, or
Y¹ represents hydroxy, and
Y² represents hydroxy, lower alkoxy or, if X represents H₂, also lower alkanoyloxy, and salts of compounds in which X represents oxo and Y² represents hydroxy, organic radicals designated "lower" containing a maximum of 7 carbon atoms.

2. A compound according to claim 1, in which R² represents methoxycarbonyl, ethoxycarbonyl or isopropoxycarbonyl.

3. A compound according to claim 1, in which the methylene radical, represented by R¹ and R² together, and/or the group 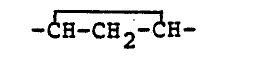 represented by the symbol -B-B- is/are ß-oriented.

4. A compound according to any one of claims 1 to 3, in which X represents oxo and Y¹ and Y2 together represent the oxygen bridge -0-.

5. An alkali metal salt of a compound according to any one of claims 1 to 3 in which X represents oxo and each of Y¹ and Y² represents hydroxy.

8. A compound according to claim 1, which is 9a,11a-epoxy-7a-methoxycarbonyl-20-spirox-4-ene-3,21-dione.

7. A compound according to claim 1, which is 9α,11α-epoxy-6β,7β-methylene-20-spirox-4-ene-3,21-dione.

8. A compound according to any one of claims 1 to 7 for use as an aldosterone-antagonistic active ingredient.

9. A process for the manufacture of steroid compounds of the formula I defined in claim 1, including salts of compounds in which X is oxo and Y² is hydroxy, characterised in that
a) a compound of the formula 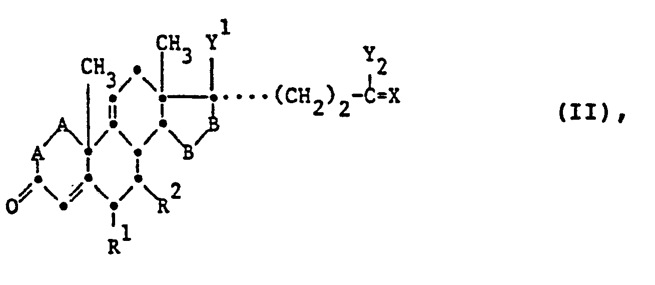 in which A-A, B-B, R¹, R², X, Y¹ and Y² have the meanings given above, is treated with a peroxy acid, or
b) a compound of the formula 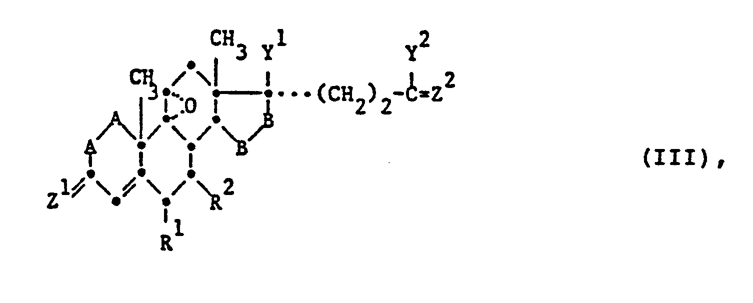 in which A-A, B-B, R¹, R², Y¹ and Y² have the meanings given above and at least one of the symbols Z^{I} and Z² represents hydroxy together with hydrogen and the other has the same meaning or represents oxo or, in the case of the symbol Z², may also represent two hydrogen atoms, is treated with an oxidising agent, or
c) for the manufacture of a compound in which Y¹ represents hydroxy and the other symbols have the meanings given above, a compound of the formula 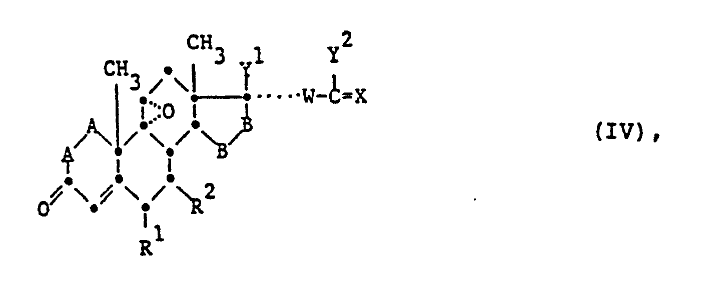 in which A-A, B-B, R¹, R², X and Y² have the meanings given above, Y¹ represents hydroxy and W represents a group -CH = CH- or -C ≡ C-, is hydrogenated to saturate the multiple bond in the side chain, or
d) for the manufacture of a compound of the formula I in which Y¹ and Y² together represent the oxygen bridge -0- and the other symbols have the meanings given above, a compound of the formula 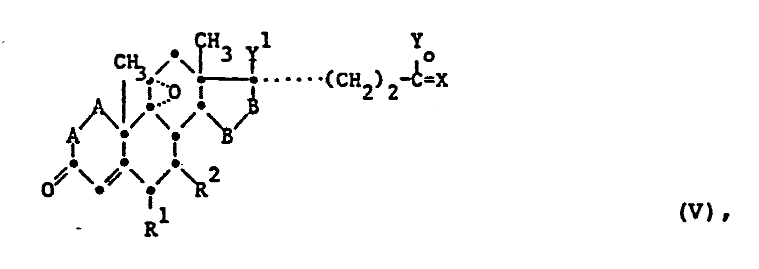 in which A-A, B-B, R¹, R² and X have the meanings given above, Y' represents hydroxy and Yₒ represents a leaving group, is cyclised with the group Yₒ being removed, or
e) for the manufacture of a compound of the formula I in which R¹ represents hydrogen and R² represents a lower alkoxycarbonyl group and the other symbols have the meanings given above, a compound of the formula 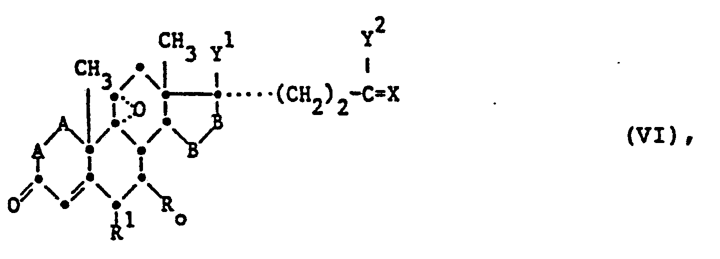 in which A-A, B-B, X, Y¹ and Y² have the meanings given above, R¹ represents hydrogen and Rₒ represents free carboxy, or a reactive derivative or salt of such a compound, is converted into an ester, or
f) for the manufacture of a compound in which R¹ and R² together represent a methylene bridge and the other symbols have the meanings given above, the methylene group is added to a compound of the formula 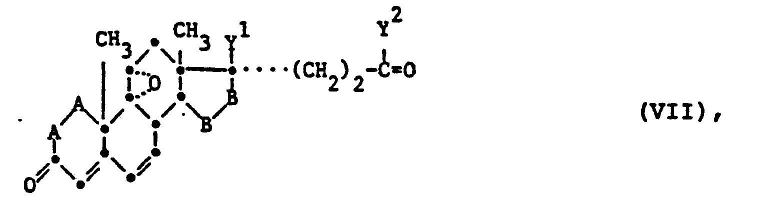 in which A-A, B-B, Y¹ and Y² have the meanings given above, and, if desired,
g) a resulting compound of the formula I in which -A-A- represents -CH₂-CH₂- is treated with a dehydrogenation agent to introduce the 1,2-double bond, and/or
h) a resulting compound of the formula I in which each of Y¹ and Y² represents a hydroxy group is cyclised by removing the elements of water to form a compound of the formula I in which Y¹ and Y² together represent the oxygen bridge, and/or
i) a resulting compound of the formula I in which X represents two hydrogen atoms is oxidised to a corresponding compound in which X represents oxo, and/or
j) in a resulting compound of the formula I in which X represents two hydrogen atoms and each of Y¹ and Y² represents a free hydroxy group the terminal hydroxy group is acylated, and/or
k) a resulting compound of the formula I in which X represents oxo, Y¹ represents hydroxy and Y² represents hydroxy or lower alkoxy, or the two together represent the oxygen bridge -0-, is converted into a salt of the corresponding 17β-hydroxy-21-carboxylic acid of the formula I in which X represents oxo and each of Y¹ and Y² represents hydroxy, and/or such a salt is converted into the free acid and/or the free acid or a salt thereof is converted into a lower alkyl ester.

10. A pharmaceutical composition containing as active ingredient one of the compounds defined in claims 1 to 8, if desired together with a diuretic which is nonspecific with regard to the excretion of electrolytes.

11. A process for the preparation of a pharmaceutical composition according to claim 10, characterised in that the active ingredient(s) defined therein is(are) incorporated with at least one pharmaceutically acceptable carrier by non-chemical means.

## Claims (Claims for the following Contracting State(s) : AT)

1. A process for the manufacture of steroid compounds of the formula I 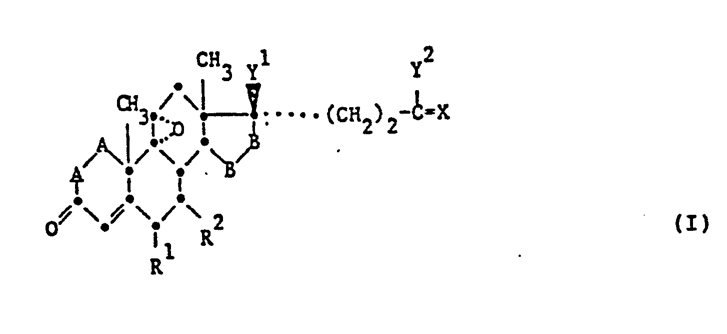 in which
-A-A- represents the group -CH₂-CH₂- or -CH=CH-,
R¹ represents hydrogen, and
R² represents an a-oriented lower alkoxycarbonyl radical, or R¹ and R² together represent an a- or β-oriented methylene radical, -B-B- represents the group -CH₂-CH₂- or an a- or β-oriented group 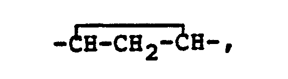
X represents two hydrogen atoms or oxo,
Y¹ and Y² together represent the oxygen bridge -0-, or
Y² represents hydroxy, and
Y² represents hydroxy, lower alkoxy or, if X represents H₂, also lower alkanoyloxy, and salts of compounds in which X represents oxo and Y² represents hydroxy, organic radicals designated "lower" containing a maximum of 7 carbon atoms, characterised in that
a) a compound of the formula 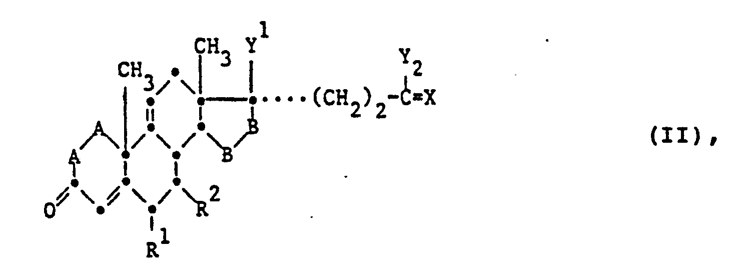 in which A-A, B-B, R¹, R², X, Y¹ and Y² have the meanings given above, is treated with a peroxy acid, or
b) a compound of the formula 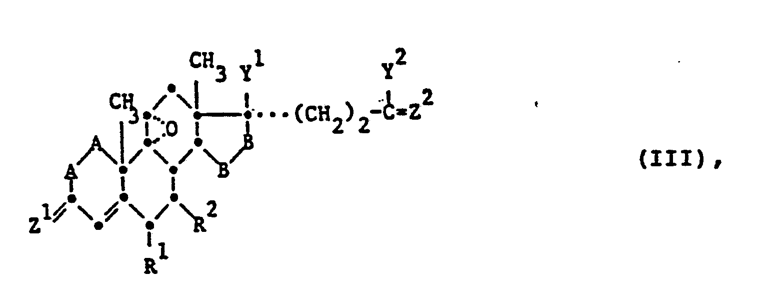 in which A-A, B-B, R¹, R², Y¹ and Y² have the meanings given above and at least one of the symbols Z¹ and Z² represents hydroxy together with hydrogen and the other has the same meaning or represents oxo or, in the case of the symbol Z², may also represent two hydrogen atoms, is treated with an oxidising agent, or
c) for the manufacture of a compound in which Y¹ represents hydroxy and the other symbols have the meanings given above, a compound of the formula 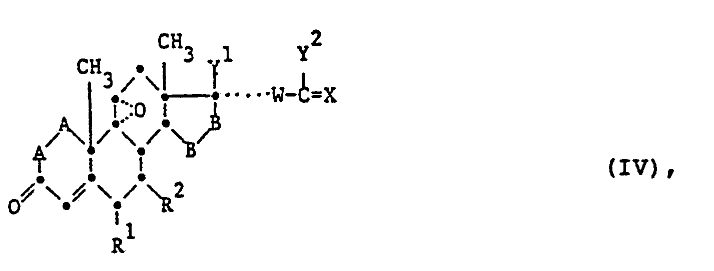 in which A-A, B-B, R¹, R², X and Y² have the meanings given above, Y¹ represents hydroxy and W represents a group -CH = CH- or -C≡C-, is hydrogenated to saturate the multiple bond in the side chain, or
d) for the manufacture of a compound of the formula I in which Y¹ and Y² together represent the oxygen bridge -0- and the other symbols have the meanings given above, a compound of the formula 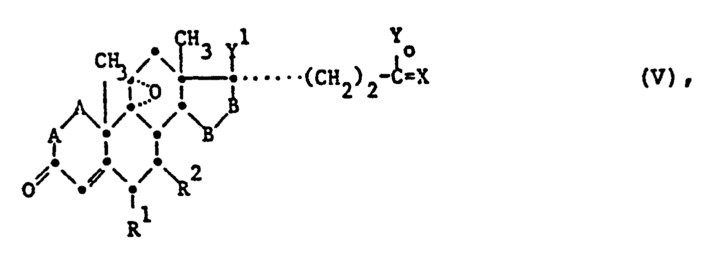 in which A-A, B-B, R¹, R² and X have the meanings given above Y¹ represents hydroxy and Yₒ represents a leaving group, is cyclised with the group Yₒ being removed, or
e) for the manufacture of a compound of the formula I in which R¹ represents hydrogen and R² represents a lower alkoxycarbonyl group and the other symbols have the meanings given above, a compound of the formula 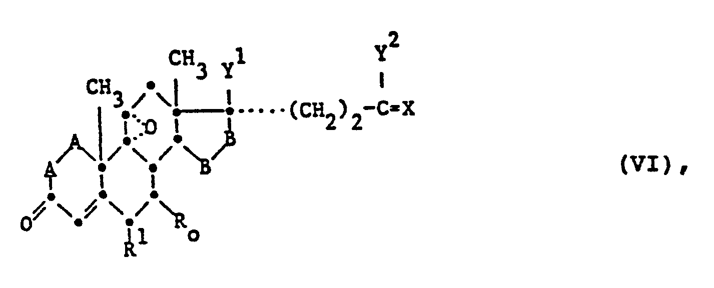 in which A-A, B-B, X, Y¹ and Y² have the meanings given above, R¹ represents hydrogen and Rₒ represents free carboxy, or a reactive derivative or salt of such a compound, is converted into an ester, or
f) for the manufacture of a compound in which R^{I} and R² together represent a methylene bridge and the other symbols have the meanings given above, the methylene group is added to a compound of the formula 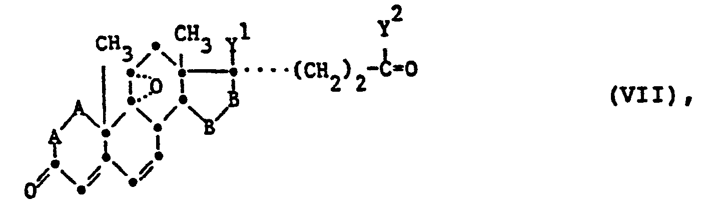 in which A-A, B-B, Y¹ and Y² have the meanings given above, and, if desired,
g) a resulting compound of the formula I in which -A-A- represents -CH₂-CH₂- is treated with a dehydrogenation agent to introduce the 1,2-double bond, and/or
h) a resulting compound of the formula I in which each of Y' and Y² represents a hydroxy group is cyclised by removing the elements of water to form a compound of the formula I in which Y' and Y² together represent the oxygen bridge, and/or
i) a resulting compound of the formula I in which X represents two hydrogen atoms is oxidised to a corresponding compound in which X represents oxo, and/or
j) in a resulting compound of the formula I in which X represents two hydrogen atoms and each of Y¹ and Y² represents a free hydroxy group the terminal hydroxy group is acylated, and/or
k) a resulting compound of the formula I in which X represents oxo, Y¹ represents hydroxy and Y² represents hydroxy or lower alkoxy, or the two together represent the oxygen bridge -0-, is converted into a salt of the corresponding 17β-hydroxy-21-carboxylic acid of the formula I in which X represents oxo and each of Y¹ and Y² represents hydroxy, and/or such a salt is converted into the free acid and/or the free acid or a salt thereof is converted into a lower alkyl ester.

2. A process according to claim 1, characterised in that there is manufactured a compound of the formula I in which R² represents methoxycarbonyl, ethoxycarbonyl or isopropoxycarbonyl.

3. A process according to claim 1, characterised in that there is manufactured a compound of the formula I in which R^{I} and R² together represent a ß-oriented methylene radical and/or -B-B- represents the β-oriented group

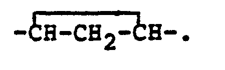

4. A process according to claim 1, characterised in that there is manufactured a compound of the formula I in which X represents oxo and Y¹ and Y² together represent the oxygen bridge -0-.

5. A process according to any one of claims 1 to 3, characterised in that there is manufactured an alkali metal salt of a compound of formula I in which X represents oxo and each of Y¹ and Y² represents hydroxy.

6. A process according to claim 4, characterised in that a potassium salt is manufactured.

7. A process according to claim 1, characterised in that 9a,11a-epoxy-7a-methoxycarbonyl-20-spirox-4-ene-3,21-dione is manufactured.

8. A process according to claim 1, characterised in that 9a,11a-epoxy-7a-isopropoxycarbonyl-20-spirox-4- ene-3,21-dione is manufactured.

9. A process according to claim 1, characterised in that 9α,11α-epoxy-6β,7β-methylene-20-spirox-4-ene-3,21-dione is manufactured.

10. A process for the preparation of a pharmaceutical composition containing as active ingredient one of the compounds defined in any one of claims 1 to 9, if desired together with a diuretic which is non-specific with regard to the excretion of electrolytes, characterised in that the active ingredient(s) is(are) incorporated with at least one pharmaceutically acceptable carrier by non-chemical means.

11. A pharmaceutical composition prepared in accordance with claim 10.

## Revendications (Revendications pour l'(les) Etat(s) contractant(s) suivant(s) : BE,CH, DE, FR, GB, IT, LI, LU, NL, SE)

1. Composé stéroide de formule 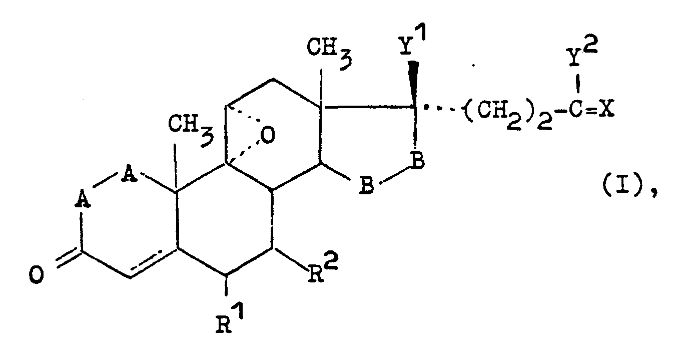 dans laquelle
-A-A- est le groupe -CH₂-CH₂- ou -CH=CH-,
R¹ est l'hydrogène et
R² est un alcoxycarbonyle inférieur a-orienté, ou
R¹ et R² représentent ensemble un reste méthylène a- ou β-orienté,
-B-B- est le groupe -CH₂-CH₂- ou un groupe a- ou ß-orienté 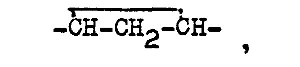
X représente deux atomes d'hydrogène ou un oxo,
Y¹ et Y² représentent ensemble le pont oxygène -0- ou
Y¹ est l'hydroxyle et
Y² est l'hydroxyle, un alcoxy inférieur ou, si X est H₂, un alcanoyloxy inférieur aussi,
ainsi que des sels de composés, dans lesquels X est un oxo et Y² est l'hydroxyle, dans lesquels les restes organiques désignés par "inférieur" contiennent au plus 7 atomes de carbone.

2. Composé selon la revendication 1, dans lequel R² représente le méthoxycarbonyle, l'éthoxycarbonyle ou l'isopropoxycarbonyle.

3. Composé selon la revendication 1, dans lequel le reste méthylène représenté par la signification commune de R¹ et R² et/ou le groupe 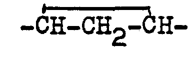 représenté par le symbole -B-B- est orienté en β.

4. Composé selon l'une des revendications 1 - 3, dans lequel X représente un oxo et Y¹ et Y² représentent ensemble le pont oxygène -0-.

5. Sel de métal alcalin d'un composé selon l'une des revendications 1 - 3, dans lequel X représente un oxo et chaque Y¹ et Y² représente l'hydroxyle.

6. Composé selon la revendication 1, qui est la 9a,11a-époxy-7a-méthoxycarbonyl-20-spiroxène-4-3,21-dione.

7. Composé selon la revendication 1, qui est la 9α,11α-époxy-6β,7β-méthylène-20-spiroxène-4-3,21-dione.

8. Composé selon l'une des revendications 1 - 7 pour être utilisé comme principe actif antagoniste de l'aldostérone.

9. Procédé de préparation des composés stéroïdes définis dans la revendication 1, de formule I, y compris les sels de ces composés, dans lesquels X est un oxo et Y² est l'hydroxyle, caractérisé en ce que
a) on traite un composé de formule 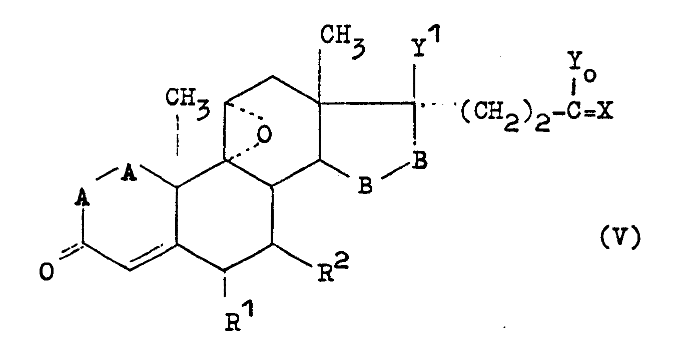
dans laquelle A-A, B-B, R¹, R² et X ont les significations données ci-dessus, Y¹ est l'hydroxyle et Yo est un groupe labile, ou
e) pour préparer un composé de formule l, dans laquelle R¹ est l'hydrogène et R² un groupe alcoxycarbonyle inférieur et les autres symboles ont les significations données ci-dessus, on transforme en ester un composé de formule 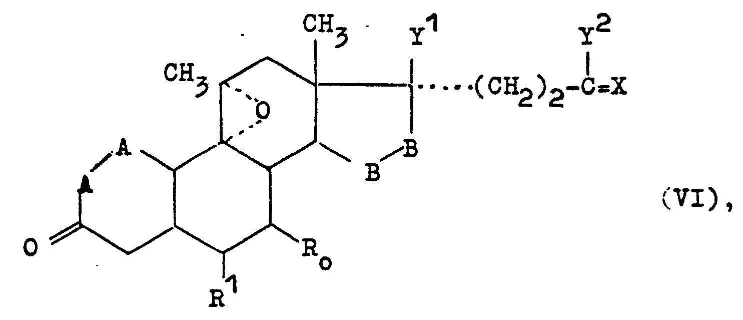 dans laquelle A-A, B-B, X, Y¹ et Y² ont les significations données ci-dessus, R¹ représente l'hydrogène et Rₒ un carboxyle libre, ou un dérivé réactif ou un sel d'un tel composé, ou
f) pour préparer un composé dans lequel R¹ et R² représentent ensemble un pont méthylène et les autres symboles ont les significations indiquées ci-dessus, on additionne le groupe méthylène sur un composé de formule 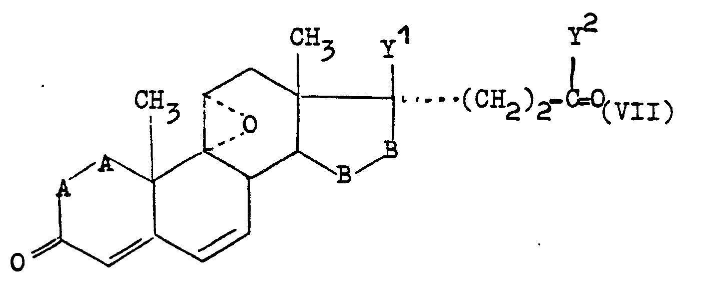 et, si c'est souhaité,
g) on traite avec un agent deshydrogénant un composé obtenu de formule I, dans lequel A-A est -CH₂-CH₂-, pour introduire la double lisison 1,2, et/ou
h) on cyclise un composé obtenu de formule l, dans lequel Y¹ et Y² représentent chacun un groupe hydroxyle, par élimination des éléments de l'eau pour donner un composé de formule l, dans laquelle Y¹ et Y² constituent ensemble le pont oxygène, et/ou
i) on oxyde un composé obtenu de formule I, dans lequel X représente deux atomes d'hydrogène en un composé correspondant, dans lequel X représente un oxo, et/ou
j) dans un composé obtenu de formule l, dans lequel X représente deux atomes d'hydrogène, et chaque Y¹ 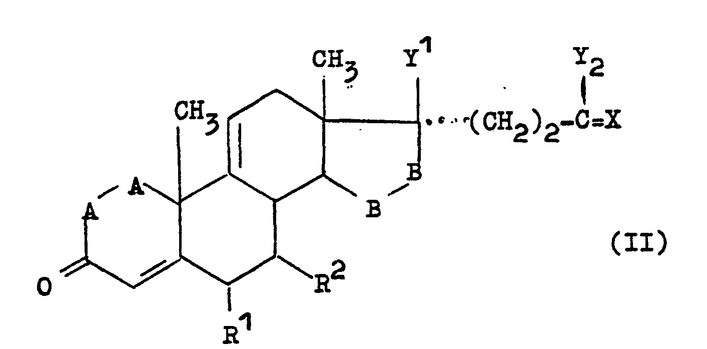 dans laquelle A-A, B-B, R¹, R², X, Y¹ et Y² ont les significations données ci-dessus, avec un peroxyacide, ou
b) on traite avec un agent oxydant un composé de formule 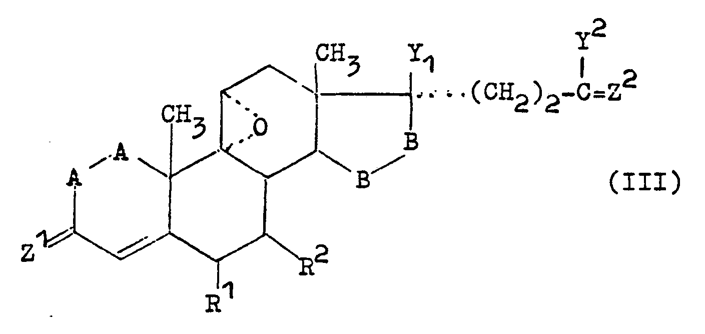 dans laquelle A-A, B-B, R¹, R², Y¹ et Y² ont les significations données ci-dessus et au moins l'un des symboles Z¹ et Z² représente l'hydroxyle avec l'hydrogène et l'autre a la même signification ou représente l'oxo, tandis que le symbole Z² peut représenter aussi deux atomes d'hydrogène, ou
c) pour préparer un composé, dans lequel Y¹ est l'hydroxyle et les autres symboles ont les significations données ci-dessus, on hydrogène jusqu'à saturation de la liaison multiple dans la chaîne latérale, un composé de formule 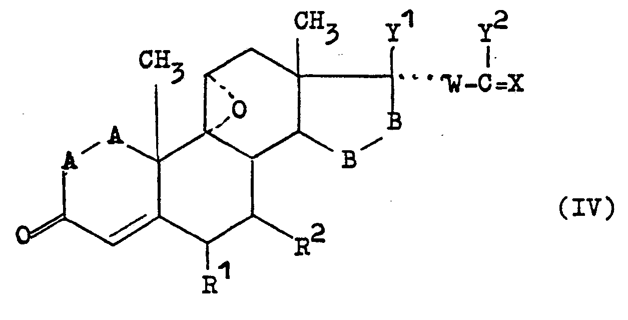 dans laquelle A-A, B-B, R¹, R², X et Y² ont les significations données ci-dessus, Y¹ représente l'hydroxyle et W un groupe -CH=CH- ou -C ≡ C- ou,
d) pour préparer un composé de formule 1 dans laquelle Y¹ et Y² ensemble représentent le pont oxygène -0-et les autres symboles ont les significations données ci-dessus, on cyclise, par élimination du groupe Y un composé de formule et Y² un groupe hydroxyle libre, ou acyle le groupe hydroxyle terminal libre, et/ou
k) on transforme un composé obtenu de formule I, dans lequel X représente un oxo, Y¹ un hydroxyle et Y² un hydroxyle ou alcoxy inférieur, ou ils représentent ensemble le pont oxygène -0-, en un sel de l'acide 17β-hydroxy-21-carboxylique de formule I, dans lequel X représente un oxo et chaque Y¹ et Y² un hydroxyle et/ou on transforme un tel sel en acide libre et/ou on transforme l'acide libre ou un de ses sels en un ester d'alkyle inférieur.

10. Composition pharmaceutique contenant comme principe actif l'un des composés définis dans les revendications 1 - 8, si c'est souhaité avec un diurétique non spécifique du point de vue de l'élimination des électrolytes.

11. Procédé de préparation d'une composition pharmaceutique selon la revendication 10, caractérisé en ce qu'on transforme le principe actif qui y est défini (les principes actifs) par des voies non chimiques avec au moins un véhicule pharmaceutiquement utilisable.

## Revendications (Revendications pour l'(les) Etat(s) contractant(s) suivant(s) : AT)

1. Procédé de préparation de composés stéroides de formule 1 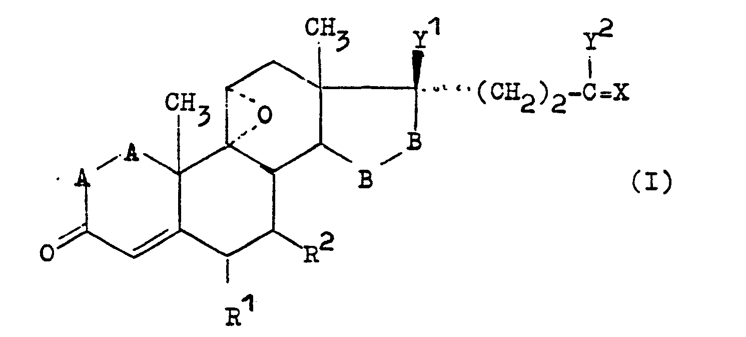 dans laquelle
-A-A- est le groupe -CH₂-CH₂- ou -CH=CH-,
R¹ est l'hydrogène et
R² est un alcoxycarbonyle inférieur a-orienté, ou
R¹ et R² représentent ensemble un reste méthylène a- ou ß-orienté,
-B-B- est le groupe -CH₂-CH₂- ou un groupe a- ou β-orienté 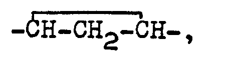
X représente deux atomes d'hydrogène ou un oxo,
Y¹ et Y² représentent ensemble le pont oxygène -0- ou
Y¹ est l'hydroxyle et
Y² est l'hydroxyle, un alcoxy inférieur ou, si X est H₂, un alcanoyloxy inférieur aussi,
ainsi que des sels des composés, dans lesquels X est un oxo et Y² est l'hydroxyle, dans lesquels les restes organiques désignés par "inférieur" présentent au plus 7 atomes de carbone, caractérisé en ce que
a) on traite un composé de formule 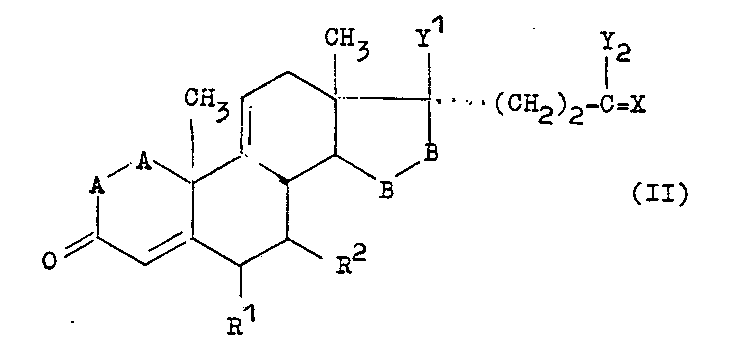 dans laquelle A-A, B-B, R¹, R², X, Y¹ et Y² ont les significations données ci-dessus, avec un peroxyacide, ou
b) on traite avec un agent oxydant un composé de formule 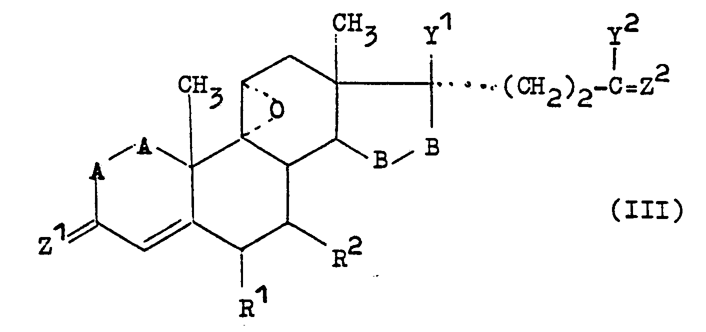 dand laquelle A-A, B-B, R¹, R², Y¹ et Y² ont les significations données ci-dessus et au moins l'un des symboles Z¹ et Z² représente l'hydroxyle avec l'hydrogène et l'autre a la même signification ou représente l'oxo, tandis que le symbole Z² peut représenter aussi deux atomes d'hydrogène, ou
c) pour préparer un composé, dans lequel Y¹ est l'hydroxyle et les autres symboles ont les significations donnés ci-dessus, on hydrogène jusqu'à saturation de la liaison multiple dans la chaîne latérale, un composé de formule 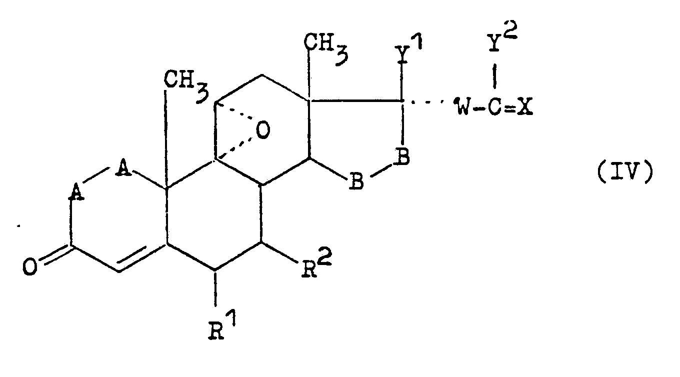 dans laquelle A-A, B-B, R¹, R², X et Y² ont les significations données ci-dessus, Y¹ représente l'hydroxyle et W un groupe -CH = CH- ou -C ≡ C- ou,
d) pour préparer un composé de formule dans laquelle Y¹ et Y² ensemble représentent le pont oxygène -0-et les autres symboles ont les significations données ci-dessus, on cyclise par élimination du groupe Yₒ un composé de formule 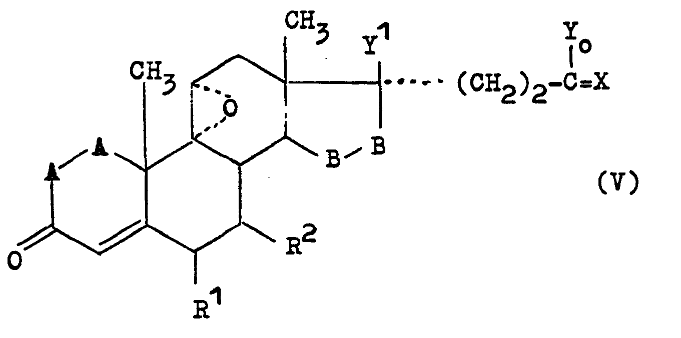 dans laquelle A-A, B-B, R¹, R² et X ont les significations données ci-dessus, Y¹ est l'hydroxyle et Yₒ est un groupe labile, ou
e) pour préparer un composé de formule I, dans laquelle R¹ est l'hydrogène et R² un groupe alcoxycarbonyle inférieur et les autres symboles ont les significations données ci-dessus, on transforme en ester un composé de formule 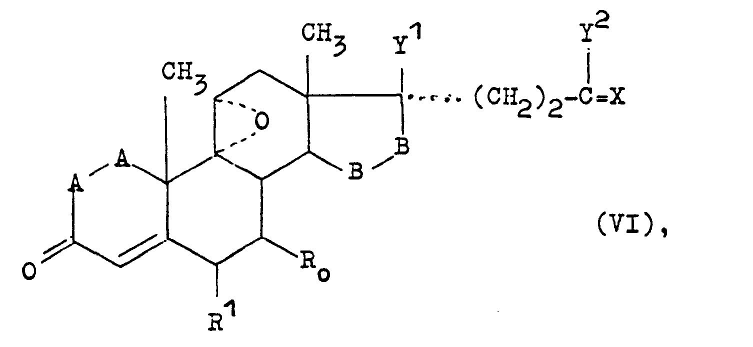 dans laquelle A-A, B-B, X, Y¹ et Y² ont les significations données ci-dessus, R¹ représente l'hydrogène et Rₒ un carboxyle libre, ou un dérivé réactif ou un sel d'un tel composé, ou
f) pour préparer un composé dans lequel R¹ et R² représentent ensemble un pont méthylène et les autres symboles ont les significations indiquées ci-dessus, on additionne le groupe méthylène sur un composé de formule 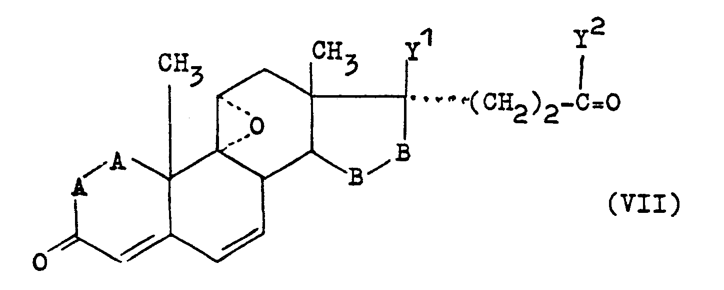 et, si c'est souhaité,
g) on traite avec un agent deshydrogénant un composé obtenu de formule I, dans lequel A-A est -CH₂-CH₂-, pour introduire la double liaison 1,2, et/ou
h) on cyclise un composé obtenu de formule 1, dans lequel Y¹ et Y² représentent chacun un groupe hydroxyle, par élimination des éléments de l'eau pour donner un composé de formule I, dans laquelle Y¹ et Y² constituent ensemble le pont oxygène, et/ou
i) on oxyde un composé obtenu de formule 1, dans lequel X représente deux atomes d'hydrogène en un composé correspondant, dans lequel X représente un oxo, et/ou
j) dans un composé obtenu de formule I, dans lequel X représente deux atomes d'hydrogène et chaque Y¹ et Y² un groupe hydroxyle libre, on acyle le groupe hydroxyle terminal libre, et/ou
k) on transforme un composé obtenu de formule I, dans lequel X représente un oxo, Y¹ un hydroxyle et Y² un hydroxyle ou alcoxy inférieur, ou ils représentent ensemble le pont oxygène -O-, en un sel de l'acide 17β-hydroxy-21-carboxylique de formule I, dans lequel X représente un oxo et chaque Y¹ et Y² un hydroxyle, et/ou on transforme un tel sel en acide libre et/ou on transforme l'acide libre ou un de ses sels en ester d'alkyle inférieur.

2. Procédé selon la revendication 1, caractérisé en ce qu'on prépare un composé de formule I, dans laquelle R² représente le méthoxycarbonyle, l'éthoxycarbonyle ou l'isopropoxycarbonyle.

3. Procédé selon la revendication 1, caractérisé en ce qu'on prépare un composé de formule I, dans laquelle R¹ et R² représentent ensemble un reste méthylène β-orienté et/ou -B-B représente le groupe 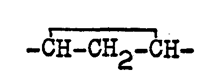 β-orienté.

4. Procédé selon la revendication 1, caractérisé en ce qu'on prépare un composé de formule I, dans laquelle X représente un oxo et Y¹ et Y² représentent ensemble le pont oxygène -0-.

5. Procédé selon l'une des revendications 1 - 3, caractérisé en ce qu'on prépare un sel de métal alcalin d'un composé de formule I, dans laquelle X représente un oxo et chaque Y¹ et Y² représente l'hydroxyle.

6. Procédé selon la revendication 4, caractérisé en ce qu'on prépare un sel de potassium.

7. Procédé selon la revendication 1, caractérisé en ce qu'on prépare la 9a,11a-époxy-7a-méthoxycarbonyl-20- spiroxène-4-3,21 -dione.

8. Procédé selon la revendication 1, caractérisé en ce qu'on prépare la 9a,11a-époxy-7a-isopropoxycarbonyl-20-spiroxène-4-3,21-dione.

9. Procédé selon la revendication 1, caractérisé en ce qu'on prépare la 9α,11α-époxy-6β,7β-méthylène-20- spiroxène-4-3,21-dione.

10. Procédé de préparation d'une composition pharmaceutique contansnt comme principe actif l'un des composés définis dans l'une des revendications 1 - 9, si c'est souhaité, avec un diurétique non spécifique du point de vue de l'élimination des électrolytes, caractérise en ce qu'on transforme le principe actif (les principes actifs) avec au moins un véhicule pharmaceutiquement utilisable.

11. Composition pharmaceutique préparée selon la revendication 10.
